# EUROPEAN PATENT APPLICATION

(11) **EP 1 792 978 A1**
(43) Date of publication of application: **06.06.2007**
(21) Application number: 05026247.6
(22) Date of filing: 01.12.2005
(51) Int. Cl.: C12N 5/06

(54) **Means and methods for the isolation and characterization of cancer stem cells**

(71) Applicant: Stiftung Caesar Center of Advanced European Studies and Research, 53175 Bonn (DE)
(72) Inventor: Royer, Hans-Dieter, 40225 Düsseldorf (DE); Brenner, Norbert, 53227 Bonn (DE)
(74) Representative: Müller Fottner Steinecke

(57) **Abstract**

Provided are assay systems for the diagnosis and treatment of cancer. More specifically, tumor stem cell-based assays for identifying therapeutic agents and targets for treating tumors are provided.

SEQUENCE LISTING

<110>  Stiftung caesar

<120>  Means and methods for the isolation and characterization of
       cancer stem cells

<130>  CA18A03/P-EP

<160>  6

<170>  PatentIn version 3.3

<210>  1
<211>  1554
<212>  DNA
<213>  homo sapiens

<220>
<221>  CDS
<222>  (114)..(1088)
<223>  Y-box binding protein (YB-1)

<400>  1
   gcggacccca gagagccctg agcagcccca ccgccgccgc cggcctagtt accatcacac      60
   cccgggagga gccgcagctg ccgcagccgg ccccagtcac catcaccgca acc atg        116
                                                              Met
                                                              1
   agc agc gag gcc gag acc cag cag ccg ccc gcc gcc ccc ccc gcc gcc       164
   Ser Ser Glu Ala Glu Thr Gln Gln Pro Pro Ala Ala Pro Pro Ala Ala
               5                   10                  15
   ccc gcc ctc agc gcc gcc gac acc aag ccc ggc act acg ggc agc ggc       212
   Pro Ala Leu Ser Ala Ala Asp Thr Lys Pro Gly Thr Thr Gly Ser Gly
           20                  25                  30
   gca ggg agc ggt ggc ccg ggc ggc ctc aca tcg gcg gcg cct gcc ggc       260
   Ala Gly Ser Gly Gly Pro Gly Gly Leu Thr Ser Ala Ala Pro Ala Gly
       35                  40                  45
   ggg gac aag aag gtc atc gca acg aag gtt ttg gga aca gta aaa tgg      308
   Gly Asp Lys Lys Val Ile Ala Thr Lys Val Leu Gly Thr Val Lys Trp
   50                  55                  60                  65
   ttc aat gta agg aac gga tat ggt ttc atc aac agg aat gac acc aag      356
   Phe Asn Val Arg Asn Gly Tyr Gly Phe Ile Asn Arg Asn Asp Thr Lys
                   70                  75                  80
   gaa gat gta ttt gta cac cag act gcc ata aag aag aat aac ccc agg      404
   Glu Asp Val Phe Val His Gln Thr Ala Ile Lys Lys Asn Asn Pro Arg
               85                  90                  95
   aag tac ctt cgc agt gta gga gat gga gag act gtg gag ttt gat gtt      452
   Lys Tyr Leu Arg Ser Val Gly Asp Gly Glu Thr Val Glu Phe Asp Val
           100                 105                 110
   gtt gaa gga gaa aag ggt gcg gag gca gca aat gtt aca ggt cct ggt      500
   Val Glu Gly Glu Lys Gly Ala Glu Ala Ala Asn Val Thr Gly Pro Gly
       115                 120                 125
   ggt gtt cca gtt caa ggc agt aaa tat gca gca gac cgt aac cat tat      548
   Gly Val Pro Val Gln Gly Ser Lys Tyr Ala Ala Asp Arg Asn His Tyr
   130                 135                 140                 145
   aga cgc tat cca cgt cgt agg ggt cct cca cgc aat tac cag caa aat      596
   Arg Arg Tyr Pro Arg Arg Arg Gly Pro Pro Arg Asn Tyr Gln Gln Asn
                   150                 155                 160
   tac cag aat agt gag agt ggg gaa aag aac gag gga tcg gag agt gct      644
   Tyr Gln Asn Ser Glu Ser Gly Glu Lys Asn Glu Gly Ser Glu Ser Ala
               165                 170                 175
   ccc gaa ggc cag gcc caa caa cgc cgg ccc tac cgc agg cga agg ttc      692
   Pro Glu Gly Gln Ala Gln Gln Arg Arg Pro Tyr Arg Arg Arg Arg Phe
           180                 185                 190
   cca cct tac tac atg cgg aga ccc tat ggg cgt cga cca cag tat tcc      740
   Pro Pro Tyr Tyr Met Arg Arg Pro Tyr Gly Arg Arg Pro Gln Tyr Ser
       195                 200                 205
   aac cct cct gtg cag gga gaa gtg atg gag ggt gct gac aac cag ggt      788
   Asn Pro Pro Val Gln Gly Glu Val Met Glu Gly Ala Asp Asn Gln Gly
   210                 215                 220                 225
   gca gga gaa caa ggt aga cca gtg agg cag aat atg tat cgg gga tat      836
   Ala Gly Glu Gln Gly Arg Pro Val Arg Gln Asn Met Tyr Arg Gly Tyr
                   230                 235                 240
   aga cca cga ttc cgc agg ggc cct cct cgc caa aga cag cct aga gag      884
   Arg Pro Arg Phe Arg Arg Gly Pro Pro Arg Gln Arg Gln Pro Arg Glu
               245                 250                 255
   gac ggc aat gaa gaa gat aaa gaa aat caa gga gat gag acc caa ggt      932
   Asp Gly Asn Glu Glu Asp Lys Glu Asn Gln Gly Asp Glu Thr Gln Gly
           260                 265                 270
   cag cag cca cct caa cgt cgg tac cgc cgc aac ttc aat tac cga cgc      980
   Gln Gln Pro Pro Gln Arg Arg Tyr Arg Arg Asn Phe Asn Tyr Arg Arg
       275                 280                 285
   aga cgc cca gaa aac cct aaa cca caa gat ggc aaa gag aca aaa gca      1028
   Arg Arg Pro Glu Asn Pro Lys Pro Gln Asp Gly Lys Glu Thr Lys Ala
   290                 295                 300                 305
   gcc gat cca cca gct gag aat tcg tcc gct ccc gag gct gag cag ggc      1076
   Ala Asp Pro Pro Ala Glu Asn Ser Ser Ala Pro Glu Ala Glu Gln Gly
                   310                 315                 320
   ggg gct gag taa atgccggctt accatctcta ccatcatccg gtttagtcat      1128
   Gly Ala Glu
   ccaacaagaa gaaatatgaa attccagcaa taagaaatga acaaaagatt ggagctgaag      1188
   acctaaagtg cttgcttttt gcccgttgac cagataaaat aactatctgc attatctatg      1248
   cagcatgggg tttttattat ttttacctaa agacgtctct ttttggtaat aacaaacgtg      1308
   ttttttaaaa aagcctggtt tttctcaata cgcctttaaa ggtttttaaa ttgtttcata      1368
   tctggtcaag ttgagatttt taagaacttc atttttaatt tgtaataaaa gtttacaact 1428
   tgattttttc aaaaaagtca acaaactgca agcacctgtt aataaaggtc ttaaataata 1488
   aaaaaaaaaa aaaaaaaaaa aaaaaaaaaa aaaaaaaaaa aaaaaaaaaa aaaaaaaaac 1548
   cctgag 1554

<210> 2
<211> 324
<212> PRT
<213> homo sapiens

<400> 2

Met Ser Ser Glu Ala Glu Thr Gln Gln Pro Pro Ala Ala Pro Pro Ala
1               5                   10                  15

Ala Pro Ala Leu Ser Ala Ala Asp Thr Lys Pro Gly Thr Thr Gly Ser
            20                  25                  30

Gly Ala Gly Ser Gly Gly Pro Gly Gly Leu Thr Ser Ala Ala Pro Ala
        35                  40                  45

Gly Gly Asp Lys Lys Val Ile Ala Thr Lys Val Leu Gly Thr Val Lys
    50                  55                  60

Trp Phe Asn Val Arg Asn Gly Tyr Gly Phe Ile Asn Arg Asn Asp Thr
65                  70                  75                  80

Lys Glu Asp Val Phe Val His Gln Thr Ala Ile Lys Lys Asn Asn Pro
                85                  90                  95

Arg Lys Tyr Leu Arg Ser Val Gly Asp Gly Glu Thr Val Glu Phe Asp
            100                 105                 110

Val Val Glu Gly Glu Lys Gly Ala Glu Ala Ala Asn Val Thr Gly Pro
        115                 120                 125

Gly Gly Val Pro Val Gln Gly Ser Lys Tyr Ala Ala Asp Arg Asn His

130                 135                 140

Tyr Arg Arg Tyr Pro Arg Arg Arg Gly Pro Pro Arg Asn Tyr Gln Gln
145                 150                 155                 160

Asn Tyr Gln Asn Ser Glu Ser Gly Glu Lys Asn Glu Gly Ser Glu Ser
                165                 170                 175

Ala Pro Glu Gly Gln Ala Gln Gln Arg Arg Pro Tyr Arg Arg Arg Arg
            180                 185                 190

Phe Pro Pro Tyr Tyr Met Arg Arg Pro Tyr Gly Arg Arg Pro Gln Tyr
        195                 200                 205

Ser Asn Pro Pro Val Gln Gly Glu Val Met Glu Gly Ala Asp Asn Gln
    210                 215                 220

Gly Ala Gly Glu Gln Gly Arg Pro Val Arg Gln Asn Met Tyr Arg Gly
225                 230                 235                 240

Tyr Arg Pro Arg Phe Arg Arg Gly Pro Pro Arg Gln Arg Gln Pro Arg
                245                 250                 255

Glu Asp Gly Asn Glu Glu Asp Lys Glu Asn Gln Gly Asp Glu Thr Gln
            260                 265                 270

Gly Gln Gln Pro Pro Gln Arg Arg Tyr Arg Arg Asn Phe Asn Tyr Arg
        275                 280                 285

Arg Arg Arg Pro Glu Asn Pro Lys Pro Gln Asp Gly Lys Glu Thr Lys
    290                 295                 300

Ala Ala Asp Pro Pro Ala Glu Asn Ser Ser Ala Pro Glu Ala Glu Gln
305                 310                 315                 320

Gly Gly Ala Glu
<210>  3
<211>  9
<212>  PRT
<213>  artificial sequence

<220>
<223>  peptide

<400>  3

Tyr Pro Tyr Asp Val Pro Asp Tyr Ala
1               5

<210>  4
<211>  9
<212>  PRT
<213>  artificial sequence

<220>
<223>  peptide

<400>  4

Tyr Asp Val Pro Asp Tyr Ala Ser Leu
1               5

<210>  5
<211>  30
<212>  DNA
<213>  artificial sequence

<220>
<223>  oligonucleotide

<400>  5
gcggccgctt caagcgtaat ctggaacgtc                                        30

<210>  6
<211>  27
<212>  DNA
<213>  artificial sequence

<220>
<223>  oligonucleotide

<400>  6
accatggagg gatcggagag tgctccc                                          27

## Description

### Field of the invention

The present invention relates to the diagnosis and treatment of cancer. More specifically, the present invention relates to tumor stem cell-based assays for identifying therapeutic agents and targets for treating cancer. The present invention further relates to the use of the tumor stem cells of the present invention for gene expression profiling and for identifying or assaying compounds, for example cytostacics, therapeutic agents, hormones, blocking compounds and for use in toxicity screening. In particular, the present invention relates to the use of the tumor stem cells of the present invention in drug discovery and microarray technology.

### Background of the invention

Cancer remains the number two cause of mortality. Despite the remarkable progress in understanding the molecular basis of cancer, this knowledge has not yet been translated into effective therapeutic strategies. In particular, breast cancer is the most common cancer in American women, with approximately one in nine women developing breast cancer in their lifetime. Unfortunately, metastatic breast cancer is still an incurable disease. Most women with metastatic breast cancer succumb to the disease. Traditional modes of therapy (radiation therapy, chemotherapy, and hormonal therapy), while useful, have been limited by the emergence of treatment-resistant cancer cells. Clearly, new approaches are needed to identify targets for treating metastatic breast cancer and cancer generally.

Thus, one major goal in cancer research is to identify the appropriate targets for therapeutic intervention. For this reason, transgenic animal models as well as xenograft animal models have already been established, wherein the transgenic and xenograft animals exhibit a phenotype which parallels the phenotype that is observed in human cancers.

However, screening methods performed on animals are cumbersome and have the disadvantage that they require the use of a large number of life mammals, in particular rats and mice, and obviously are not amenable to high throughput screening.

Thus, there is a need for cell-based in vitro assay systems that can be easily performed and give reliable results. The solution to said technical problem is achieved by providing the embodiments characterized in the claims, and described further below.

### Summary of the invention

The current cancer therapies do not address the core of the disease, leading to high rates of "relapse". One reason is the investigation of the wrong cells. The isolation of cancer stem cells derived from the cancerous tumor solves the problem. The pharmaceutical industry is highly interested in testing of compounds and agents on the right cells to find more effective drugs for cancer therapy. For those tests, the tumor stem cells provided by the present invention are highly convenient and appropriate.

In particular, the present invention provides non-human tumor stem cells which allow to much more effectively focus the development of anti-tumor therapeutics and diagnostics. More specifically, the tumor stem cells of the present invention are derived from a tumor of a non-human animal, wherein said tumor is induced by the presence of or exposure to a tumor inducing agent. Most preferably said tumor inducing agent is a foreign nucleic acid molecule which is preferably derived from a tumor promoting gene. Furthermore, the present invention relates to continuous cell lines of the isolated tumor stem cells.

The provision of tumor stem cells in accordance with the present invention for the first time enables identification of differences between those tumor cells which are most likely responsible for metastases, i.e. tumor stem cells, and "normal" stem cells. These differences can be used for designing drug therapy in the treatment of cancer more specifically and to avoid severe side effects on normal stem cells and somatic cells.

The present invention provides in vitro cell-based assays of tumor stem cell function as well as methods for using tumor stem cells to identify factors influencing tumor stem cell proliferation, for analyzing the gene expression patterns or protein expression patterns, to identify new anti-cancer drug targets, to predict the sensitivity of tumors that have been developed because of the presence of a particular oncogene to existing anti-cancer treatment regimens, to model anti-cancer treatment, to test new therapeutic compounds, to identify and test new diagnostic markers, to treat tumors, to produce genetically modified tumor stem cells, to prepare differentiated cells and tissue from the tumor stem cells of the present invention and to prepare cDNA libraries and microarrays of polynucleotides and polypeptides from tumor stem cells.

Hence, the investigation of the isolated cancer stem cells can reveal new targets for cancer therapy, and therewith new agents can be developed. Furthermore, new proteins may be revealed, which enable the prediction of clinical courses of cancer as well as of effective therapies. Thus, diagnostic tests can be developed and sold or provided as a service.

The investigation of the isolated cancer stem cells by for example microarray analysis can lead to gene signatures, i.e. cancer profiles enabling clinically relevant predictions. Thus, the present invention relates to investigations on the level of gene expression, but also to custom-arrays, comprising said signatures.

Furthermore, the present invention relates to the use of transgenic animals which have been genetically engineered to develop a tumor upon expression of a human oncogene for the isolation of stem cells from said tumor. In addition, the present invention relates to in vivo methods for assaying tumor stem cells of the present invention in a non-human animal into which said tumor stem cell has been introduced, optionally after said tumor stem cell has been treated by for example chemical agents and/or has been further genetically engineered.

In particular, the present invention relates to the isolation, cultivation and investigation of cancer stem cells derived from YB-1 transgenic non-human animals. YB-1 is an oncogene, which is mostly overexpressed in tumors and which correlates with a high relapse rate. Additionally, it could be shown that despite a unique origin in YB-1 transgenic mice different tumor types are generated. The tumor stem cell system of the present invention is particularly useful for the investigation of oncogenes such as YB-1, which are directly or indirectly involved in mediating chemosensitivity and -resistance of tumors, which is one of the biggest clinical problems encountered in the treatment of cancer.

Furthermore the tumor stem cells of the present invention can be used to characterize expression profiles associated with a particular cancer useful in diagnosing and monitoring the course of disease in a patient, in determining gene targets for intervention, and in testing novel treatment regimens.

Thus, the findings obtained in accordance with the present invention pave the way for deeper understanding of tumor development and offer new approaches for diagnosis and treatment of cancerous diseases.

### Brief description of the drawings

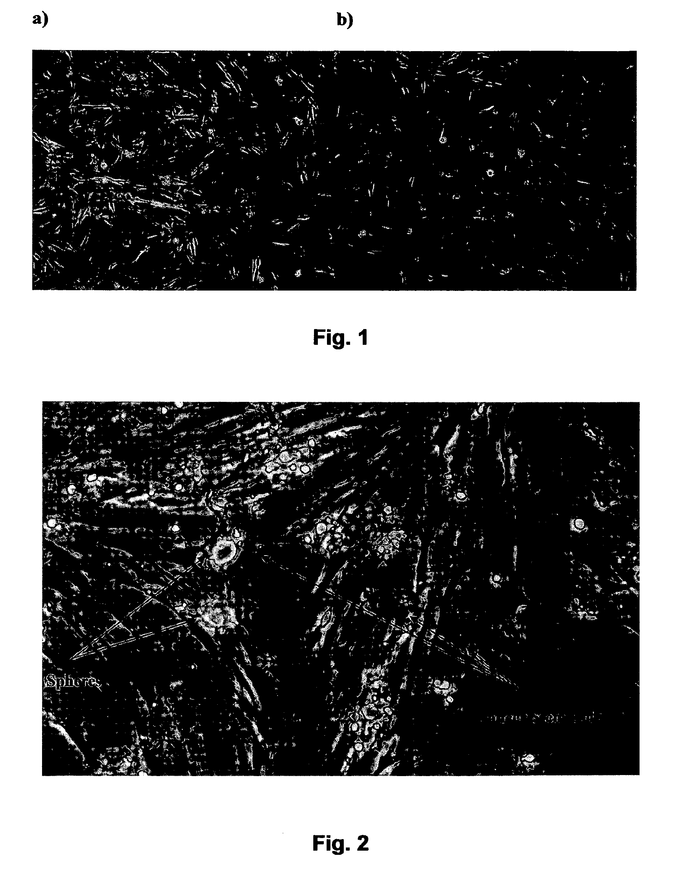

- **Fig.1:**: illustrates cell lines established by seeding tumor-derived cancer cells into a culture medium composed of DMEM low glucose/RPMI (1:1), containing stable glutamine (Biochrom) and 10 % FBS, wherein a) and b) correspond to the cancer cells isolated from a tumor of two different mice, herein referred to as mouse one and mouse two, respectively. The mentioned tumors in both mice were induced by the overexpression of human YB-1 gene; see also Example 1. The figure shows the heterogenic but predominantly fibroblastoidal morphology of the cell lines, containing only few suspension cells. Microscopy was carried out at a magnification of 50.

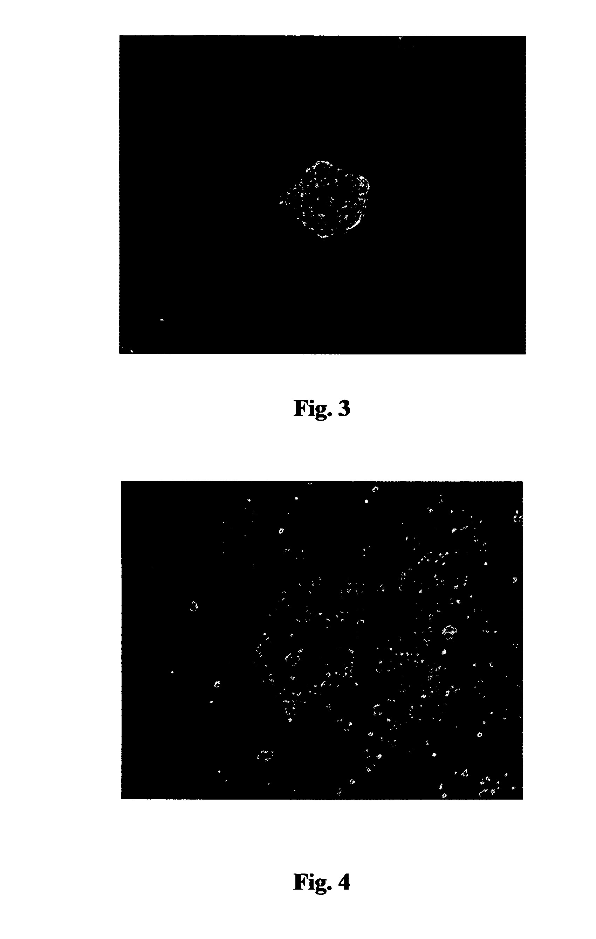

- **Fig. 2:**: illustrates the enrichment of cancer stem cells, obtained from the tumor of mouse one. It especially shows the enrichment of small cancer stem cells after centrifugation, discarding of the supernatant, and resuspension of the cells in fresh medium. It is shown that the cancer stem cells also grow partially in spherical forms. The arrows indicate the cancer stem cells and spheres, respectively. Microscopy was carried out at a magnification of 50.
- **Fig. 3:**: illustrates the formation of spheres for cells obtained from the tumor which were cultured in another medium (besides the medium described hereinbefore), composed of DMEM containing 20 % serum replacement (Invitrogen), different cytokines and a combination of bFGF (10 ng/ml) and LIF (1000 U/ml). Microscopy was carried out at a magnification of 200.
- **Fig. 4:**: illustrates cell cultures of mouse two, 18 days after addition of prolactin, dexamethasone and insulin (PDI) at a ratio of 1 µg/ml: 10⁻⁷ M: 10 µg/ml to the medium to induce differentiation. A massive increase in sphere formation of small suspension cells in the culture 18 days after induction is shown. Microscopy was carried out at a magnification of 630.

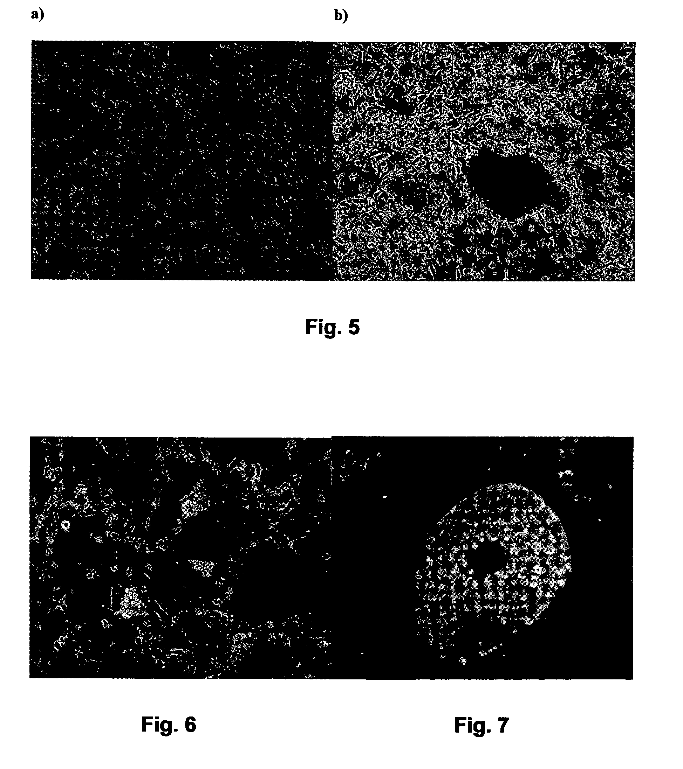

- **Fig. 5:**: illustrates a cell culture of mouse one, 18 days after addition of prolactin, dexamethasone and insulin in a ratio of 1 µg/ml: 10⁻⁷ M: 10 µg/ml to the medium for differentiation purposes. It also shows a massive increase in sphere formation of small suspension cells in the culture 18 days after induction, with microscopy being carried out at a magnification of 100 in a) and one month after induction and with microscopy being carried out at a magnification of 50 in b), respectively.
- **Fig. 6:**: illustrates the development of additional cells with a strong fat production. A culture of mouse two and there especially one area with a high accumulation of fat-producing cells is shown. Microscopy was carried out at a magnification of 100.
- **Fig. 7:**: illustrates a differentiated fat-producing cell, developed in the cell culture of mouse one after induction of differentiation by the addition of prolactin, dexamethasone and insulin at the hereinbefore mentioned ratio. Microscopy was carried out at a magnification of 630.

### Definitions

For the purposes of this description, the term "stem cell" can refer to either stem cell or germ cell, for example embryonic stem (ES) and germ (EG) cell, respectively, but also including adult stem cells and tumor stem cells. Minimally, a stem cell has the ability to proliferate and form cells of more than one different phenotype, and is also capable of self-renewal, either as part of the same culture, or when cultured under different conditions. Embryonic stem cells are also typically telomerase positive and OCT-4 positive. Telomerase activity can be determined using TRAP activity assay (Kim et al., Science 266 (1997), 2011), using a commercially available kit (TRAPeze(R) XK Telomerase Detection Kit, Cat. s7707; Intergen Co., Purchase N.Y.; or TeloTAGGG(TM) Telomerase PCR ELISAplus, Cat. 2,013,89; Roche Diagnostics, Indianapolis). hTERT expression can also be evaluated at the mRNA level by RT-PCR. The LightCycler TeloTAGGG(TM) hTERT quantification kit (Cat. 3,012,344; Roche Diagnostics) is commercially available for research purposes.

Furthermore, stem cells may be defined as self-renewing cells that undergo symmetric and asymmetric divisions to self-renew or differentiate into multiple kinds of differentiated progeny (Lin and Schagat, Trends Genet. 13 (1997), 33-39; Morrison et al., Cell 88 (1997), 287-298; Bums and Zon, Dev. Cell 3 (2002), 612-613). There are at least three types of stem cells: Totipotent, pluripotent and multipotent. A single totipotent stem cell can grow into an entire organism. Pluripotent stem cells cannot grow into a whole organism, but they can become any other cell of a particular germ layer, such as ectoderm, mesoderm or endoderm. Multipotent (also referred to as unipotent) stem cells can become any cells of a given tissue derived from one of the germ layers.

"Pluripotent" refers to cells that retain the developmental potential to differentiate into a wide range of cell lineages including the germ line. The terms "embryonic stem cell phenotype" and "embryonic stem cell-like cell" also are used interchangeably herein to describe cells that are undifferentiated and thus are pluripotent cells and that preferably are capable of being visually distinguished from other adult cells of the same animal.

"Progenitor cells" as used herein, refers to cells that are not fully mature or differentiated, but are less developmentally primitive than stem cells and which are progeny of a less differentiated stem cell. In contrast to stem cells, progenitor cells are not believed to be typically indefinitely self-replicating and, functionally, are not typically capable of long-term clonogenicity in immunodeficient animals in xenograft assays.

"Cancer stem cells" and "tumor stem cells", as defined herein, are cells with properties very similar to "normal" stem cells, exhibiting the most important property of self-renewal. Various cancers include cancer stem cells, rare cells with indefinite potential for self-renewal, that drive tumorigenesis. Cancer stem cells represent a small population of cells in tumors that resemble adult stem cells, the self-replicating cells that give raise to specialized cells in tissues. Like "normal" stem cells, cancer stem cells can seed the growth of new tissue, in this special case of new cancers, since it has been shown that implantation of a few tumor stem cells into mice led to the generation of all the cells of the original tumor; see for example Al-Hajj et al., Proc. Natl. Acad. Sci. USA published online February 24, 2003; Reya et al., Nature 414 (2001), 105-111; Miyamoto et al., Proc. Natl. Acad. Sci. USA 97 (2000), 7521-7526.

In accordance with the present invention the tumor stem cells are preferably characterized by their potency to be differentiated in vitro into a pre-selected cell type.

The term "tumor" is commonly understood as an abnormal mass of tissue. Tumors are a classic sign of inflammation, and can be benign or malignant (cancerous). There are dozens of different types of tumors. Their names usually reflect the kind of tissue they arise in, and may also tell something about their shape or how they grow. For example, a medulloblastoma is a tumor that arises from embryonic cells (a blastoma) in the inner part of the brain (the medulla). Diagnosis depends on the type and location of the tumor. Tumor marker tests and imaging may be used; some tumors can be seen (for example, tumors on the exterior of the skin) or felt (palpated with the hands). Treatment is also specific to the location and type of the tumor. The term "cancer" refers to any malignant tumor, i.e. carcinomas and sarcomas and involves the uncontrolled growth of abnormal cells that have mutated from normal tissues, for example epithelial or connective tissue. Unless stated otherwise the terms "tumor" and "cancer" are used interchangeably herein.

Sarcomas comprise for example fibrosarcoma, malignant fibrous hystiocytoma, dermatofibrosarcoma, liposarcoma, rhabdomyosarcoma, leiomyosarcoma, hemangiosarcoma, Kaposi's sarcoma, lymphangiosarcoma, synovial sarcoma, neurofibrosarcoma, extrasceletal chondrosarcoma, extraskeletal osteosarcoma, osteosarcoma and chondrosarcoma.

Epithelial cancer consists of big cells being very similar to the natural epithelium of the body. It mostly develops in those parts which are provided with epithelium like for example skin or mucosa or for example the lips. Said form of cancer does not spread rapidly nor produces secondary growth in other organs compared to other types of cancer, but also affects the lymphatic glands. Epithelial cancer comprises colorectal cancer, gastrointestinal cancer, non-small cell lung cancer and cancer of the liver. One prominent example for epithelial cancer is ovarian epithelial cancer.

Breast cancer is the most common cancer in women, but metastatic breast cancer is still incurable and as a consequence most women with metastatic breast cancer succumb to the disease. Despite advances in detection and treatment of metastatic breast cancer, mortality from this disease remains high because current therapies (radiation therapy, chemotherapy, and hormonal therapy) are limited by the emergence of therapy-resistant cancer cells. As a result, metastatic breast cancer remains an incurable disease using current treatment strategies.

The term "tumor gene" or "oncogene" is a commonly understood as a unit of nucleic acid molecule that normally directs cell growth, but which can also promote or allow the uncontrolled growth of cancer if damaged (mutated) by an environmental exposure to carcinogens, or damaged or missing because of an inherited defect. An oncogene is a gene that when mutated or expressed at abnormally-high levels contributes to converting a normal cell into a cancer cell. Cancer cells are cells that are engaged in uncontrolled mitosis. In context of the present invention the term "tumor promoting gene" includes genes of any kind, which are directly or indirectly involved in the development of tumors in a mammal, most preferably in humans. Unless stated otherwise, the terms "tumor gene" and "oncogene" are used interchangeably herein.

A "foreign nucleic acid molecule" is a nucleotide sequence that is foreign to the cell, vector or position in the genome, wherein it is placed. Unless stated otherwise, the terms "foreign", "heterologous" and "exogenous" are used interchangeably herein. The term "heterologous" is used broadly in this aspect to indicate that the gene/sequence of nucleotides in question have been introduced into said cells of the animal or an ancestor thereof, using genetic engineering, i.e. by human intervention. For example, a heterologous gene may be additional to a corresponding endogenous gene. Nucleic acid molecules heterologous, exogenous or foreign to an animal cell may be non-naturally occurring in cells of that type, tissue, race or species. Thus, the heterologous nucleic acid molecule may comprise a coding sequence of or derived from a particular type of animal cell, animal race or species, placed within the context of an animal cell of a different type, race or species. In some embodiments, the foreign nucleic acid molecule may not be the ultimate nucleic acid molecule of interest but a regulatory element which is introduced into target animal cell such as to control and/or modulate the expression an endogenous gene or the activity of its encoded product. These embodiments are particularly suited for the investigation and exploitation of signal transduction pathways. For the purpose of the present invention, said "foreign nucleic acid molecule" is preferably a human nucleic acid molecule which directly or indirectly induces, promotes or supports tumor growth.

Y box binding protein 1 (YB-1), previously termed "nuclease sensitive element binding protein 1 (NSEP-1)" is also known as, YB1, DBPB, MDR-NF1, BP-8, CSDB and CSDA2. Nucleotide and amino acid sequences of the human YB-1 gene as well as further information and corresponding publications can be retrieved from public databases such as GenBank hosted by the National Center for Biotechnology Information (NCBI) and EMBL hosted by the European Molecular Biology Laboratory (EMBL) through the European Bioinformatics Institute [EBI], which one of the central suppliers of biology data services for both the academic and industrial research and development. The most frequently used tools are SMART, STRING, ELM and Harvester, which can be used in order to easily retrieve sequence information of a given gene and its putative orthologues. In particular, reference is made to GenBank Accession Numbers NM_004559 [gi:34098945] and UniProt-EBI Primary accession number P67809 [secondary accession numbers P16990, P16991]. The nucleotide and amino acid sequence of YB-1 are exemplified in SEQ ID NO: 1 and 2, respectively. The YB-1 gene and its characterization have first been described in Didier et al., Proc. Natl. Acad. Sci. USA 85 (1988), 7322-7326; Sakura et al., Gene 73 (1988), 499-507 and Kolluri and Kinniburgh, Nucleic Acids Res. 19 (1991) 4771. Isolation of YB-1 cDNA clones by binding site screening of a Hela expression library using a human papillomavirus type 18 enhancer oligonucleotide has been described by the group of Royer et al. in Spitkovsky et al., Nucleic Acids Res. 20 (1992), 797-803. Furthermore, tissue restricted expression and chromosomal localization of the YB-1 gene is described. Moreover, information on the biological activity, tissue distribution and the signal transduction pathway YB-1 is thought to be involved in can be retrieved by using the Bioinformatic Harvester© of the EMBL. In this respect, it will be understood that through these means further appropriate tumor inducing or promoting genes are provided, which in accordance with the present invention can be used to program a non-human animal cell so as to become tumorigenic.

"Differentiation" is the process whereby relatively unspecialized cells (e.g. stem cells) acquire specialized structural and/or functional features characteristic of mature cells. Similarly, "differentiate" refers to this process. Typically, during differentiation, cellular structure alters and tissue-specific proteins appear.

The term "enrich" as used herein refers to increasing the quantity of tumor stem cells, and in particular embodiments it refers to increasing the ratio of tumor stem cells to non-tumor stem cells. This may be further described as successively isolating tumor stem cells from undesired cells. Usually, the ratio of tumor stem cells to non-tumor cells increases by a factor 2, preferably 5 and most preferably by a factor of more than 10. The enriched population of tumor stem cells of the present invention is preferably substantially free of progenitor cells. Most preferably, the tumor stem cells of the present invention are characterized by pluripotency or multipotency; see supra.

The terms "nucleic acid", "polynucleotide" and "nucleic acid sequence" refer to a polymer of nucleotides of any length. Included are genes and gene fragments, mRNA, tRNA, rRNA, ribozymes, cDNA, recombinant polynucleotides, branched polynucleotides, plasmids, vectors, isolated DNA and RNA, nucleic acid probes, and primers. As used in this disclosure, the term polynucleotides refer interchangeably to double- and single-stranded molecules. Unless otherwise specified or required, any embodiment of the invention that is a polynucleotide encompasses both a double-stranded form, and each of the two complementary single-stranded forms known or predicted to make up the double-stranded form. As such, the nucleic acid sequence can, for example, include one or more exons, with or without, as appropriate, introns, as well as one or more suitable control sequences. In one example, the nucleic acid molecule contains a single open reading frame which encodes a desired nucleic acid product. The nucleic acid sequence is operably linked to a suitable promoter. A nucleic acid sequence encoding a desired nucleic acid product can be isolated from nature, modified from native sequences or manufactured de novo, as described in, for example, Ausubel et al., Current Protocols in Molecular Biology, John Wiley & Sons, New York (1998); and Sambrook et al., Molecular Cloning: A Laboratory Manual, 2nd edition, Cold Spring Harbor University Press, New York(1989). Nucleic acids can be isolated and fused together by methods known in the art, such as exploiting and manufacturing compatible cloning or restriction sites. Included are nucleic acid analogs such as phosporamidates and thiophosporamidates.

A cell is said to be "genetically altered", "transfected", "genetically transformed" or "transgenic" when a polynucleotide has been transferred into the cell by any suitable means of artificial manipulation or where the cell is a progeny of the originally altered cell that has inherited the polynucleotide. The polynucleotide will often comprise a transcribable sequence encoding a protein of interest, which enables the cell to express the protein at an elevated level. The term "transformed" cells includes stably transformed cells in which the inserted DNA is capable of replication either as an autonomously replicating plasmid or as part of the host chromosome, as well as transiently transformed cells which express the inserted DNA or RNA for limited periods of time. The genetic alteration is said to be "inheritable" if progeny of the altered cell have the same alteration.

In the context of encoding sequences, promoters, and other genetic elements, the term "heterologous" indicates that the element is derived from a genotypically distinct entity from that of the rest of the entity to which it is being compared. For example, a promoter or gene introduced by genetic engineering techniques into an animal of a different species is said to be a heterologous polynucleotide. An "endogenous" genetic element is an element that is in the same place in the chromosome where it occurs in nature, although other elements may be artificially introduced into a neighboring position.

The term "operably linked", as used herein, is defined to mean that the gene (or the nucleic acid sequence) is linked to control sequences in a manner which allows expression of the gene (or the nucleic acid sequence). General, operably linked means contiguous.

The terms "polypeptide", "peptide" and "protein" are used interchangeably in this disclosure to refer to polymers of amino acids of any length. The polymer may comprise modified amino acids, it may be linear or branched and it may be interrupted by non-amino acids.

As used herein, the term "microarray" refers to cell based arrays, nucleic acid or nucleotide arrays or protein or peptide including antibody arrays that can be used to detect biomolecules, for instance to measure gene expression. "Array, "bioarray", "microarray", "nylon filter", "slide," and "chip" are used interchangeably in this disclosure. Various kinds of arrays are made in research and manufacturing facilities worldwide, some of which are available commercially. There are, for example, two main kinds of nucleic acid arrays that differ in the manner in which the nucleic acid materials are placed onto the array substrate: spotted arrays and in situ synthesized arrays. One of the most widely used oligonucleotide arrays is GeneChip™ made by Affymetrix, Inc. The oligonucleotide probes that are 20- or 25-base long are synthesized in silico on the array substrate. These arrays tend to achieve high densities (e.g., more than 40,000 genes per cm²). The spotted arrays, on the other hand, tend to have lower densities, but the probes, typically partial cDNA molecules, usually are much longer than 20- or 25-mers. Representative types of spotted cDNA arrays include LifeArray made by Incyte Genomics and DermArray made by IntegriDerm (or Invitrogen). Pre-synthesized and amplified cDNA sequences are attached to the substrate of these kinds of arrays. Protein and peptide arrays also are known; see, e.g., Zhu et al., Science 293 (2001), 2101. The term "microarray", as used herein, also includes high-density arrays of cells and tissues, in particular comprising stem cells for examining complex biological interactions simultaneously, which are identified by a specific location on a slide array. For example, a scanning microscope detects the bound, labeled sample and measures the visualized probe to ascertain the activity of the genes of interest in genotyping, cellular studies and expression analysis. It is a powerful technology that allows the simultaneous measurement of expression levels for up to tens of thousands of genes. Usually microarrays are used to simultaneously study large numbers of genes and their regulation, by probing with labeled nucleic acids taken from biological samples.

Microarray data, as used herein, encompass any data generated using various arrays, including but not limited to the nucleic acid arrays described above. Typical microarray data include collections of gene expression levels measured using nucleic acid arrays on biological samples of different biological states and origins. The methods of the present invention may be employed to analyze any microarray data; irrespective of the particular nucleic acid microarray platform (e.g., nylon filters, glass slides, plastic, or silicon chips) from which the data are generated.

Gene expression, as used herein, refers in general to the transcription from DNA sequences into RNA molecules, which encode certain proteins with structural, enzymatic, or regulatory functions. The expression level of a given gene measured at the nucleotide level refers to the amount of RNA transcribed from the gene measured on a relevant or absolute quantitative scale, and in general refers to the relative abundance of the accumulated mRNA transcript. The expression level of a given gene measured at the protein level refers to the amount of protein translated from the transcribed RNA measured on a relevant or absolute quantitative scale. The measurement can be, for example, an optical density value of a fluorescent or radioactive signal, on a blot or a microarray image. Differential expression, as used herein, means that the expression levels of certain genes, as measured at the RNA or protein level, are different between biological samples in different states, tissues, or type of cells. Differential expression may also be observed relative to a reference standard. Such standard may be determined based on the context of the expression experiments, the biological properties of the genes under study, and/or statistical significance criteria.

Expression analysis, differential display techniques and target gene identification also comprise assaying proteins of the tumor stem cells of the present invention compared to a control such a corresponding non-tumor stem cell or normal stem cell. For example, proteins isolated from an enriched population of tumor stem cells or isolated tumor cells can be separated by two-dimensional polyacrylamide gel electrophoresis (2D-PAGE) systems. Two-dimensional gel electrophoresis is well-known in the art and typically involves iso-electric focusing along a first dimension followed by SDS-PAGE electrophoresis along a second dimension; see, e.g., Hames et al, Gel Electrophoresis of Proteins: A Practical Approach (IRL Press, New York (1990); Lander, Science 274 (1996), 536-539. The resulting electrophoretograms can be analyzed by numerous techniques, including mass spectrometric techniques, western blotting and immunoblot analysis using polyclonal and monoclonal antibodies, and internal and N-terminal micro-sequencing. Using these techniques, it is possible to identify a substantial fraction of all the proteins produced under given physiological conditions, including in cells (e.g., in tumor stem cells) exposed to a drug, or in cells modified by, e.g., deletion or over-expression of a specific gene.

Hence, so-called proteomics is a rapidly emerging scientific discipline that holds great promise in identifying novel diagnostic and prognostic biomarkers for human cancer; see, e.g., review by Ornstein and Petricoin, Oncology 18 (2004), 521-532. Technologic improvements have made it possible to profile and compare the protein composition within defined populations of cells, i.e. the tumor cells of the present invention and "normal" stem cells. Improvements in 2D-PAGE technology have greatly enhanced resolution and sensitivity providing a more reproducible and comprehensive survey. Image analysis software and robotic instrumentation have been developed to facilitate comparisons of complex protein expression patterns and isolation of differentially expressed protein spots. Differential in-gel electrophoresis (DIGE) facilitates protein expression by labeling different populations of proteins with fluorescent dyes. Isotope-coded affinity tagging (ICAT) uses mass spectroscopy for protein separation and different isotope tags for distinguishing populations of proteins.

Reverse-phase protein arrays offer a robust new method of quantitatively assessing expression levels and the activation status of a panel of proteins. Surface-enhanced laser-desorption/ionization time-of-flight (SELDI-TOF) mass spectroscopy rapidly assesses complex protein mixtures. Furthermore, 2-D liquid mapping methods can be used for the classification of tumor stem cells. For example, chromatofocusing (CF) and nonporous (NPS) RP-HPLC separation of ovarian serous carcinoma cell line lysates has been performed continuously using an integrated protein fractionation system ProteomeLab™ PF 2D (Beckman Coulter, Inc. Fullerton, CA, USA); see Wang et al., in MCP Papers, published online on September 2, 2005 as Manuscript T500023-MCP200. The Beckman Coulter ProteomeLab PF 2D system may be used in accordance with the manufacturer's Use Protocol. Combined with artificial intelligence-based pattern recognition algorithms, this emerging technology can generate highly accurate diagnostic information.
The design and analysis of experiments with high throughput biological assay data including analysis of proteomics and metabolomics data by mass spectrometry and NMR spectroscopy is reviewed by Rocke, Semin. Cell. Dev. Biol. 15 (2004), 703-713. Examples for the proteotypic classification of tumors in transgenic mouse models of mammary neoplasia (mice overexpressing Neu (Erbb2), Hras, Myc, Notch4, SV40-TAg, Tgfa, and Wnt1) have recently been described by Mikaelian et al., Breast Cancer Res. 6 (2004), R668-679.
It is to be understood that all the above referenced methods may be adapted and applied to the tumor stem cells of the present invention as well.

Simple ratio, as used herein, with respect to a gene, is the ratio of its hybridization intensity measured from a first sample or a first group of samples to its hybridization intensity measured from a second sample or a second group of samples. The first and second samples or groups of samples may be from different types of cells, or they may correlate with different biological and/or pathological states, according to various embodiments of this invention. The hybridization intensities may be normalized before the ratio is calculated according to certain embodiments, to account for the background noise, the bias introduced by the different samples, among other things.

The foregoing methods are preferably conducted at least twice on a given sample using at least one different primer pair specific for a specific marker gene. Following detection, one may compare the results seen in a given stem cell with a statistically significant reference group of reference stem cells. In this way, it is possible to correlate the number and kind of markers with various clinical states, toxicity values, or disease prognosis.

As used herein, "profile" or "profiling" of a cell, in particular tumor stem cell or chemical composition or compound refers to a pattern of alterations in gene or protein expression, or both, or physiological properties in a normal stem, tumor cell, cancer stem cell, tissue, etc. contacted by the chemical composition compared to a like cell or tissue in contact only with culture medium.

If not stated otherwise the terms "compound", "substance" and "(chemical) composition" are used interchangeably herein and include but are not limited to therapeutic agents (or potential therapeutic agents), growth factors, lineage commitment factors, food additives and nutraceuticals, agents of known toxicities such as neurotoxins, hepatic toxins, toxins of hematopoietic cells, myotoxins, carcinogens, teratogens, or toxins to one or more reproductive organs. The chemical compositions can further be agricultural chemicals, such as pesticides, fungicides, nematicides, and fertilizers, cosmetics, including so-called "cosmeceuticals", industrial wastes or by-products, or environmental contaminants. They can also be animal therapeutics or potential animal therapeutics. Compounds to be screened may also be obtained from diversity libraries, such as random or combinatorial peptide or non-peptide libraries. Many libraries are known in the art that can be used, e.g., chemically synthesized libraries, recombinant (e.g., phage display libraries), and in vitro translation-based libraries.

### General Techniques

For further elaboration of general techniques useful in the practice of this invention, the practitioner can refer to standard textbooks and reviews in cell biology, tissue culture, embryology, and tumor biology.

With respect to tissue culture and embryonic stem cells, reference can be made to Teratocarcinomas and embryonic stem cells: A practical approach (Robertson, ed., IRL Press Ltd. 1987); Guide to Techniques in Mouse Development (Wasserman et al. eds. , Academic Press 1993); Embryonic Stem Cell Differentiation in Vitro (Wiles, Meth. Enzymol. 225 (1993), 900); Properties and uses of Embryonic Stem Cells: Prospects for Application to Human Biology and Gene Therapy (Rathjen et al., Reprod. Fertil. Dev. 10 (1998), 31).

General methods in molecular and cellular biochemistry can be found in such standard textbooks as Molecular Cloning: A Laboratory Manual, 3rd Ed. (Sambrook et al., Harbor Laboratory Press 2001); Short Protocols in Molecular Biology, 4th Ed. (Ausubel et al. eds., John Wiley & Sons 1999); Protein Methods (Bollag et al., John Wiley & Sons 1996); Non-viral Vectors for Gene Therapy (Wagner et al. eds., Academic Press 1999); Viral Vectors (Kaplitt & Loewy eds., Academic Press 1995); Immunology Methods Manual (Lefkovits ed., Academic Press 1997); and Cell and Tissue Culture: Laboratory Procedures in Biotechnology (Doyle & Griffiths, John Wiley & Sons 1998). Reagents, cloning vectors and kits for genetic manipulation referred to in this disclosure are available from commercial vendors such as BioRad, Stratagene, Invitrogen, Sigma-Aldrich, and ClonTech.

### Detailed description of the invention

The present invention generally relates to tumor stem cells that have the capacity for extensive proliferation, the ability to give rise to a tumor in vivo, and which are capable of being differentiated in vitro into selected cell types. The present invention is based on the discovery that stem cells can be isolated from designer tumors in non-human animals which have been induced to develop the tumor upon the presence of a human oncogene.

In particular, the present invention is based upon the surprising finding that cells with an established tumor induced by the expression of the human oncogene Y box binding protein 1 (YB-1) have the properties of stem cells. More particularly, in accordance with the present invention, it could be demonstrated that within cells obtained from tumors of human oncogene transgenic mice a small percentage of the suspended cells have the capacity for extensive proliferation and in vitro differentiation opposed to adherent cells which only barely show proliferative activity. As further demonstrated by the examples, the suspension cells can be further enriched and cultivated in vitro; see Example 2. Moreover, it could be demonstrated that these cells can be differentiated in vitro similarly like "normal" stem cells; see Example 3.

Most recently, the role of YB-1 in breast cancer has been investigated in transgenic mice where expression of a human hemagglutinin (HA-tagged) YB-1 cDNA was controlled by the ovine β-lactoglobulin promoter (BLG); see the publication by Bergman et al., Cancer Res. 65 (2005), also published online, the disclosure content of which is incorporated herein by reference. The transgenic animals developed multiple mammary tumors which were diagnosed as invasive breast carcinomas with different histological types. While breast carcinoma cells from the BLG-YB-1 breast tumors were cultured and analyzed to be aneuploid and display the phenotype of ongoing numerical chromosomal instability in tissue culture, this publication failed to envisage the possibility that said breast carcinoma cells include at least a small fraction of cancer stem cells which behave like "normal" stem cells and can be differentiated in vitro.

One critical feature in the discovery of tumor stem cells in accordance with the present invention was the finding that suspension cells derived from tumor tissue comprise stem cells, which can be isolated, enriched, cultivated and differentiated like normal stem cells. Hitherto, only the tumorigenic properties of populations of tumor cells have been described, probably due to the fact that the tumor cell populations previously investigated were substantially comprised of other high proliferating cells, such as lineage committed progenitor cells. Hence, although cancer stem cell theory has been proposed in the recent literature, a corresponding proof was missing and the implications of this theory were discussed controversially.

Thus, the present invention for the first time provides tumor stem cells which are derived from a tumor of a non-human animal, wherein said tumor is induced by a tumor inducing agent, foreign to said animal. In principle, the tumor inducing agent may be of any kind as long as it has a tumor-inducing capability. Examples for tumor inducing agents are radiation, viruses, chemicals, organic solvents, as well as other stimuli described further below, which are well known to those skilled in the art. However, in accordance with the present invention it is preferred that the tumor is induced by the presence of a foreign nucleic acid molecule, which is preferably derived from a tumor promoting gene. As mentioned, the present invention is based on the use of a YB-1 transgenic animal/tumor stem cell system for the investigation of developing tumors. In accordance with the present invention, tumor stem cells are obtainable from transgenic animals overexpressing YB-1 and can be precisely investigated, leading to an understanding of the processes in tumor cells and thus enabling their systematic abatement. Current anti-cancer therapies using cytostatics are unspecific and cause a lot of problems, since especially stem cells and other faster dividing cell types are also affected. A detailed understanding of tumor stem cell biology will allow a specific strategy, sparing the non-tumorigenic cells in case of a therapy.

Hence, the transgenic animal/tumor stem cell system of the present invention has major advantages compared to the transgenic animal models of the prior art. It is not only much easier to handle, but also enables the enrichment of pure cancer stem cell to an almost unlimited amount, and allows screening methods to be performed in high throughput format. Besides the facilitated handling of the tumor stem cells of the present invention, they can also be established and propagated as continuous tumor stem cell lines, can be genetically modified, analyzed for the presence or absence of certain (surface) markers, different proteins, protein expression patterns, sensitivity to drugs or other external stimuli like for example radiation, with the goal of identifying tumor specific targets and drugs.

International application WO02/12447 claims isolated cells from solid tumors, wherein said cells express the cell surface marker CD44 and at a lower level CD24 compared to non-tumorigenic cancer cells derived from the solid tumor. In this respect, it is observed that such isolated cells from the tumor are tumorigenic and therefore may be called "solid tumor stem cell". However, as can be inferred from the detailed description and the examples of international application WO02/12447, the so-called "solid tumor stem cells" have not actually been characterized as stem cells. In fact, it has merely been disclosed that tumorigenic cells isolated from breast cancer have proliferative and tumorigenic properties most likely because of the aberrant expression of notch-4. Furthermore, in one preferred embodiment the cancer stem cells of the present invention do not substantially express the surface marker CD44, contrary to the cells disclosed in international application WO02/12447.

Similarly, in subsequent international application WO03/050502 the isolation of tumorigenic CD44⁺CD24^{-/low} LINEAGE- cells is described, which give rise to new tumors. However, as the stem cells according to the present invention preferably do not substantially express the surface marker CD44, the present invention in one preferred embodiment does not relate to CD44⁺CD24^{-/low} LINEAGE⁻ tumorigenic cells specifically described in international applications WO02/12447 and WO03/050502.

US patent application US 2005/0022259 A1 inter alia relates to methods for identifying stem cells and/or cancer cells in transgenic non-human animals by detecting the expression of a reporter transgene. This US application however does not disclose cancer stem cells in accordance with the present invention. Furthermore, the teaching of US 2005/0022259 A1 would not allow the detection and isolation of cancer stem cells since it merely relies on a cell cycle-specific expression of a reporter gene and therefore would stain any high-proliferating cell.

International application WO2005/057172 claims the identification and isolation of leukemic stem cells by fluorescence-activated cell sorting (FACS) after staining the cells with lineage marker-specific antibodies. As already mentioned hereinbefore, FACS analysis and cell sorting usually does not result in a substantially enriched population of stem cells since almost always committed progenitor cells, i.e. non-stem cells will be identified and isolated as well. Accordingly, the present invention does not make use of the teaching of international application WO2005/057172, the embodiments disclosed therein therefore being excluded from the scope of the present invention unless they have been modified and adapted in accordance with the teaching provided in the present application.

In contrast, the population of tumor stem cells provided by the present invention is substantially free of non-stem cells. In particular, the population of tumor stem cells of the present invention will preferably have less than 25 %, more preferably less than 20 %, most preferably less than 10 % and ideally less than 5 % committed progenitor cells such as granulocyte/macrophage progenitors and megacaryocyte/erythrocyte progenitors.

Furthermore, the tumor stem cells of the present invention are characterized by the presence of a foreign nucleic acid molecule which is responsible for the tumorigenic properties of the cells. In addition, most preferably the tumor stem cells of the present invention can be characterized by their multi- or pluripotency, thus their capacity of being differentiated into the desired cell type in vitro or in vivo. Furthermore, a correlation could be found of a particular high CD133 expression to a particular low Hoechst signal. Since CD133 is an early stem cell marker, the presence of this marker in suspension cells may prove the presence of tumor stem cells. However, the presence of high CD133 expression alone might be not sufficient to conclude that the putative tumor stem cells indeed parallel the properties of normal stem cells. Therefore, the tumor stem cells of the present invention have also been characterized by applying Hoechst dye that is commonly used to identify stem cells. Since stem cells show an increased efflux of the Hoechst dye, it is used for characterization of cells and to prove their stem cell identity, see also Example 4. Two of the closely related bis-benzimides are commonly used: Hoechst 33258 and Hoechst 33342. Both dyes are excited by ultraviolet light at around 350 nm and both emit blue/cyan fluorescence light around an emission maximum at 461 nm.

In principle, any tumor may be used as the source for the isolation of the tumor stem cell of the present invention. Besides the fact that the YB-1 animal/tumor stem cell system alone already provides a source for various kinds of tumors other designer tumor animals may be used.
For example, transgenic mice which develop high-grade prostatic intraepithelian neoplasia (PIN) upon expression of the human proto-oncogene c-myc under regulatory control of the prostate-specific probasin promoter, are described in international application WO2004/000010, the disclosure content of which is incorporated herein by reference. Furthermore, a transgenic non-human animal, which overexpresses the human Ci homolog of GLI 1 and which leads to the development of tumors resembling human basal cell carcinoma (BCC) as well as other hair follicle-derived neoplasias is described in international application WO01/56376.

Transgenic animals which have been genetically engineered to develop a tumor and which therefore may be used as a source for the isolation of cancer stem cells in accordance with the present invention are known in the art; see, e.g., the reviews by Ostrand-Rosenberg in Curr. Opin. Immunol. 16 (2004), 143-150; Hoffman-Goetz, Med. Sci. Sports Exerc. 35 (2003), 1828-1833 and Van Dyke and Jacks, Cell 108 (2002), 135-144. For example, transgenic mice in which tumors arise spontaneously have been developed for most cancers. Usually, the one of three technologies is used: tissue-specific promoters to drive expression of SV40 large T antigen or tissue-specific oncogenes; deletion of tumor suppressor genes by gene targeting; or, conditional deletion of tumor suppressor genes or activation of oncogenes via Cre-lox technology. Of course, any one of these methods and transgenic mice may be used in accordance with the teaching of the present invention as well.

For example, designer tumors in mice can be produced employing a general method based on Cre-mediated recombination that can be used for ubiquitous or tissue-specific expression of protein products, including tumor-inducing oncoproteins; see for review Politi et al., Oncogene 23 (2004), 1558-1565. Further non-human animals, in particular mice which may serve as a source for isolation of tumor stem cells in accordance with present invention are described in the literature. For example, a further transgenic mouse prostate cancer model is described in Gabril et al., Gene Ther. 9 (2002), 1589-1599. A transgenic NM23-Null/SV40 mouse with hepatocellular carcinoma is described in Boissan et al., J. Natl. Cancer Inst. 97 (2005), 836-845. In addition, Tchou-Wong et al., Am. J. Respir. Cell Mol. Biol. 27 (2002), 186-193, describe the lung-specific expression of a dominant-negative mutant p53 in transgenic mice leading to an increase of spontaneous and benzo(a)pyrene-induced lung cancer. Furthermore, transgenic pigs expressing human decay-accelerating factor regulated by the porcine MCP gene promoter are reported in Murakami et al., Mol. Reprod. Dev. (2002), 302-311. Brandt et al., Oncogene 19 (2000), 2129-2137, describe the mammary gland specific hEGF receptor transgene expression which induces neoplasia and inhibits differentiation.
Thus, the present invention can be practiced using tissue or primary cells of any vertebrate species. Included are cells from human as well as non-human primates, domestic animals, livestock, and other non-human mammals, in particular rodents such as mice and rat, most preferably mice.

Although the transgenic animal/tumor stem cell system of the present invention is generally applicable to any kind of tumor it will be recognized that especially for the investigation of solid tumors the provision of tumor stem cells provides a considerable step forward in the understanding of tumor progression and development of new therapeutic approaches. This is because in contrast to for example leukemia, such as chronic myelogenous leukemia (CML) from which populations of hematopoetic cells mainly consisting of high proliferating progenitor cells can be isolated and investigated for self-renewal, the make up of solid tumors is quiet different and less understood. Accordingly, most advantageously tumor stem cells are provided y the present invention from a solid tumor such as sarcoma cell or epithelial cancer, most preferably from breast cancer or an ovarian cancer.

In principal, the tumor of the non-human animal may be induced by any foreign nucleic acid molecule the presence of which directly or indirectly results in the formation of a tumor. Usually the foreign nucleic acid molecule will be derived from a tumor promoting gene, for example an oncogene or mutated tumor suppressor gene.

While the transgenic animal/tumor stem cell system of the present invention may be applied to any mammal and thus has also utility in veterinary research, the major goal is of course the diagnosis and treatment of human cancer patients. Accordingly, in a preferred embodiment of the present invention said foreign nucleic acid molecule used to induce the tumor in the non-human animal is preferably derived from a human gene or a human pathogen.
Especially in cancer research and cancer-related drug-development there is nowadays an increased focus on oncogenes and signal transduction pathways in the context of their potential as targets for cancer therapy. Oncogenes may be grouped within seven categories, comprising growth factors, tyrosine kinases, intermediate signaling molecules, transcription factors, cell cycle regulators, DNA damage repair genes, and genes involved in apoptosis (Zhang et al., Am. J. Pharmacogenomics 5 (2005), 247-257; Zhang et al., Am. J. Pharmacogenomics 5 (2005), 327-338). Another type of gene, involved in signal transduction and execution of mitogenic signals are proto-oncogenes, usually acting through their protein products. Upon activation, it (or its product) becomes a tumor-inducing agent (oncogene). This activation comprises mutations within a proto-oncogene, loss of regulation, or an increase in protein concentration, caused by an increase of protein expression (through misregulation), an increase of protein stability which prolongs its existence. Human pathogens such as retroviruses are also known to harbor oncogenes or proto-oncogenes.

Most preferably, the tumor stem cells of the present invention are derived from a tumor animal model resembling the heterogeneity of human tumors and generating as much tumor types as possible. Advantageously, the designer tumor animal parallels the situation in human tumors, so that investigations also of the isolated tumor cells are transferable onto the human system. These conditions are optimally fulfilled by the transgenic YB-1 animal model which in accordance with the present invention served as the first example for the isolation of cancer stem cells of transgenic animals, further demonstrating that stem cells are indeed the cause of cancer diseases.

In accordance with the present invention, the YB-1 animal/tumor stem cell system is particularly suited, since the overexpression of YB-1 induces changes in the glandula morphology and an abnormal proliferation of the breast epithelium originates. Furthermore, focal hyperplasias and premalignant lesions develop in the breast glands. Furthermore, it could be observed that overexpression of YB-1 leads to the amplification of centrosomes and a progressive chromosomal instability in the breast cancer cells. The same observations could be made in human tumors. In addition, several transgenic animals expressing YB-1 developed histologically different breast tumors, all of them invasive, within 12 to 15 months.

Another advantage of using the YB-1 animal/tumor stem cell system in accordance with present invention is that YB-1 is overexpressed in approximately 75 % of human breast carcinomas. It is located on chromosome 1p34, which shows multiple copies in approximately 80 % of all breast cancer cases (Fehm et al., Breast Cancer Res. Treat. 75 (2002), 227-239). YB-1 in human tumors is associated with the intrinsic gene expression of the MDR1 gene, the gene product of which (P-glycoprotein) plays an important role in multiresistant tumors (Bargou et al., Nat. Med. 3 (1997), 447-450; Gottesmann et al., Nat. Rev. Cancer 2 (2002), 48-58). A clinical study showed that YB-1 can predict the tumor resistance and the etiopathology, independently of other clinical relevant tumor factors as for example HER2, urokinase-type plasminogen activator and plasminogen activator inhibitor-1 (Janz et al., Int. J. Cancer 97 (2002), 278-282).

A special advantage in the use of the YB-1 animal/tumor stem cell system in accordance with the present invention is that YB-1 reflects the whole variety of tumor types in humans, especially concerning breast cancer. Furthermore, a high YB-1 level correlates with a high resistance of the tumors towards the current therapy and therefore also with a bad clinical course.
For example, increased amounts of YB-1 were found in the pancreas adenocarcinoma, metastatic prostate carcinoma, ovarian carcinoma, medulloblastoma (Rhodes et al., Neoplasia 6 (2004), 1-6), malignant melanoma (Hipfel et al., Br. J. Cancer 82 (2000), 1149-1157), breast cancer (Bargou et al., Nat. Med. 3 (1997), 447-450, Janz et al., Int. J. Cancer 97 (2002), 278-282), osteosarcoma, colorectal carcinoma, lung cancer (Kohno et al., Bioassays 25 (2003), 691-698), thyroid neoplasia (Ito et al., Pathol. Int. 53 (2003), 429-433) and in prostate cancer (Gimenez-Bonafe et al., Prostate 59 (2004), 337-349). Many clinical studies showed that an increased protein amount of YB-1 negatively influences the clinical course of breast cancer (Janz et al., Int. J. Cancer 97 (2002), 278-282), ovarian carcinoma (Huang et al., Gynecol. Oncol. 93 (2004), 287-291), "non small cell" lung cancer (Gessner et al., Eur, Respir. J. 23 (2004), 14-19) and synovial carcinoma (Kohno et al., Bioassays 25 (2003), 691-698).

This led to the conclusion that YB-1 is a clinical relevant molecule which is maybe causally linked to the development of various malignant diseases.

Usually the foreign nucleic acid molecule is operably linked to an expression control sequence in order to direct cell and/or tissue specific expression of the tumor promoting agent; see also Example 1. In this respect, also inducible expression systems may be used.
In a preferred embodiment of the present invention, the nucleic acid molecule is fused to a sequence encoding a 9-residue carboxy-terminal influenza A virus haemagglutinin (HA) epitope tag; see also Example 1. Epitope tagging is a powerful and versatile strategy for detecting and purifying proteins expressed by cloned genes. To utilize this feature, protein expression vectors are typically engineered with a nucleotide sequence that encodes the peptide epitope tag. The gene of interest is cloned in-frame relative to the tag and, upon expression, the protein of interest is synthesized as a fusion protein with the peptide tag. Fusion protein detection and/or purification is mediated by highly specific antibodies to the engineered peptide, thus eliminating the need for antibodies to proteins from each newly cloned gene. Other commonly used epitope tags include glutathione-S-transferase (GST), c-myc, 6-histidine (6X-His), FLAG, green fluorescent protein (GFP), maltose binding protein (MBP), β-galactosidase, and GAL4, or any combination thereof.

The foreign nucleic acid molecule which is responsible for the development of the tumor may be present in the animal either extra chromosomally or stably integrated into the genome of the animal, preferably such that it is inherited. Extra chromosomal nucleic acid molecules may be present for example due to the infection of the animal with a virus or otherwise application of exogenous nucleic acid molecules such as viral vectors or plasmid DNA. Nevertheless, in accordance with the present invention preferably transgenic tumor animals are used. Thus, in a preferred embodiment of the present invention the foreign nucleic acid molecule is stably integrated into the genome of the tumor stem cell.

It is to be understood that in accordance with the present invention also transgenic animals can be employed that are transgenic for more than the transgene inducing the tumor. For example, a transgenic animal may contain stably integrated into its genome reporter gene constructs that provide a selection system for transgenic cells. Furthermore, transgenic animals, which express a reporter gene under the control of a promoter that is specifically expressed in tumor cells can be employed, thus providing an indication to the practitioner where a tumor is being developed and thus can be used as a source for the tumor stem cells of the present invention. A corresponding transgenic animal system is described for example in US patent application US 2004/0139487 A1.

The present invention also relates to a method for enriching a population of cells for tumor stem cells, comprising the steps of dissociating a tumor as defined hereinbefore to form a cell suspension; and culturing said cell suspension under conditions appropriate for maintenance and/or proliferation of stem cells; see Example 2. In general, embryonic stem cell technology, especially ES culture media and culture conditions may be employed. Particularly suitable methods for the enrichment, isolation, culturing and differentiation of tumor stem cells are disclosed by applicant in co-pending European patent application "Cell culture system for the enrichment and expansion of cancer stem cells", (Attorney docket No. CA18A02/P-EP), filed on the same day as the present application, i.e. on December 01, 2005. Thus, for the purpose of isolating and culturing tumor stem cells of the present invention, the disclosure content of this co-pending European patent application is incorporated herein by reference, in particular Examples 2, 5 and 6.
Naturally, the method of enriching tumor stem cells may further comprise isolating the (proliferated) tumor stem cell, and optionally introducing at least one selected cell to a culture medium that supports the growth and/or differentiation of tumor stem cells; as well as proliferating the selected cell in the culture medium. In this context, the proliferating/differentiating tumor stem cell may be contacted with a test compound; and analyzed for the effect of said test compound.

Once tumor stem cells are isolated, enriched and stably cultivated in accordance with the methods of the present invention, cell lines of said stem cells may be established according to methods known in the art. Hence, the present invention also relates to populations of tumor stem cells of the present invention. The tumor stem cell progeny of the present invention may be cryopreserved until they are needed by any method known in the art. The cells may be suspended in an isotonic solution, preferably a cell culture medium, containing a particular cryopreservant. Such cryopreservants include for example dimethylsulfoxide (DMSO), glycerol and the like and cells are frozen gradually to a temperature of approximately -150 °C.

Of course, it is envisaged by the present invention to administer or graft a tumor stem cell of the present invention or derivatives thereof to non-human animals, preferably mice, in order to investigate for example their tumorigenic potential.

In one embodiment of the method of the present invention, said tumor stem cells are genetically engineered, i.e. they are transgenic stem cells. This embodiment is particularly suited if the tumor stem cells are intended to be exposed to in vitro differentiation in order to deplete populations of differentiated cells of relatively undifferentiated cells and/or of cells of undesired cell types. This can be achieved by using a selection system that is lethal to the undesired cells and cell types, i.e. by expressing a selectable marker gene that renders cells of a specific cell type resistant to a lethal effect of an external agent, under control of a regulatory sequence that causes the gene to be preferentially expressed in the desired cell type and/or at a certain stage of development. To accomplish this, the cells are genetically altered prior to the process used to differentiate the cells into the desired lineage, in a way that the cells comprise a selectable marker and/or reporter gene operably linked to a regulatory sequence specific for the desired cell type.

Of course, the tumor stem cells used in accordance with the present invention may also be transgenic because of other reasons, for example they express a putative therapeutically active protein and/or they have been genetically altered in order to suppress an inherited disease. Those engineered stem cells may also be employed to deliver gene mouse models of various diseases like for example cancer diseases. The effect of said engineered cells used in gene therapy and "produced" as described hereinbefore can be due to (over)expression of a (mutated) gene, knock-down of gene (e.g. by RNA interference; RNAi), knock-out, or knock-in of a gene.
Any suitable expression vector for the purposes of the present invention can be used. Suitable viral vector systems for producing altered tumor stem cells according to this invention can be prepared using commercially available virus components. The introduction of the vector construct or constructs into the tumor stem cells occurs in a known manner, e.g. by transfection or with the help of viral vectors. Viral vectors comprising effector genes are generally described in the publications referenced to in the last section. Alternatively, vector plasmids can be introduced into cells by electroporation, or using lipid/DNA complexes. Exemplary is the formulation Lipofectamine 2000(TM), available from Gibco/Life Technologies. Another exemplary reagent is FuGENE(TM) 6 Transfection Reagent, a blend of lipids in non-liposomal form and other compounds in 80 % ethanol, obtainable from Roche Diagnostics Corporation. Furthermore, nucleofection of human embryonic stem cells has recently been reported by Nix et al., Stem Cells and Development 14 (2005), 378-383.

Resistance genes per se are known, for example nucleoside and aminoglycoside-antibiotic-resistance genes for, e.g. puromycin, streptomycin, bleomycin, neomycin, gentamycin or hygromycin. Further examples for resistance genes are dehydrofolate-reductase which confers a resistance against aminopterine and methotrexate, as well as multi drug resistance genes which confer a resistance against a number of antibiotics, e.g. against vinblastin, doxorubicin and actinomycin D.

It may be desirable that the cells have the ability to replicate in certain drug screening applications, and to provide a reservoir for the generation of in vitro differentiated cells such as breast cells and their precursors. The cells of this invention can optionally be telomerized to increase their replication potential, either before or after they progress to restricted developmental lineage cells or terminally differentiated cells. ES cells that are telomerized may be taken down the differentiation pathway described earlier; or differentiated cells can be telomerized directly.

Cells are telomerized by genetically altering them via transfection or transduction with a suitable vector, homologous recombination, or other appropriate technique, so that they express the telomerase catalytic component (TERT), typically under a heterologous promoter that increases telomerase expression beyond what occurs under the endogenous promoter. Particularly suitable is the catalytic component of human telomerase (hTERT), provided in international application WO98/14592. For certain applications, species homologs like mouse TERT can also be used; see international application WO99/27113. Transfection and expression of telomerase in human cells is described in Bodnar et al., Science 279 (1998), 349, and Jiang et al., Nat. Genet. 21 (1999), 111. In another example, hTERT clones are used as a source of hTERT encoding sequence, and spliced into an EcoRl site of a PBBS212 vector under control of the MPSV promoter, or into the EcoRl site of commercially available pBABE retrovirus vector, under control of the LTR promoter; see also international application WO98/14592. They can then be assessed for hTERT expression by RT-PCR, telomerase activity (TRAP assay), immunocytochemical staining for hTERT, or replicative capacity; see also supra.

Continuously replicating colonies will be enriched by further culturing under conditions that support proliferation, and cells with desirable phenotypes can optionally be cloned by limiting dilution. Depending on the intended use of the cells, other methods of immortalization may also be acceptable, such as transforming the cells with DNA encoding myc, the SV40 large T antigen, or MOT-2; see for example US patent No. 5,869,243 and international applications WO97/32972 and WO01/23555.

In another embodiment of the invention, one or more tumor stem cells or cell lines thereof are differentiated by contacting with a differentiation agent, and a composition is prepared comprising differentiated cells; see also Example 3. After the tumor stem cells have been propagated continuously in culture, differentiation can be promoted into a wide variety of cell types, including cells found in each of the endoderm, mesoderm, and ectoderm germ layers. With the appropriate combinations of growth and differentiation factors, however, cell differentiation can be controlled. Hence, the tumor stem cells of the present invention can be differentiated in vitro and in vivo into various cell types and progenitors thereof, including but not limited to hematopoietic cells, neuronal cells, pancreatic cells, hepatocyte precursor cells, adipocytes, chondrocytes, osteoblasts, retinal pigment epithelial cells, fibroblasts, skin cells such as keratinocytes, dendritic cells, hair follicle cells, renal duct epithelial cells, smooth and skeletal muscle cells, testicular progenitors, and vascular endothelial cells. Embryonic stem cell differentiation models for cardiogenesis, myogenesis, neurogenesis, epithelial and vascular smooth muscle cell differentiation in vitro have been generally described in Guan et al., Cytotechnology 30 (1999), 211-226. In vitro differentiated cardiomyocytes, neural cells, hepatocytes, adipocytes, skeletal muscle cells, vascular endothelial cells and osteoblasts are also described in US patent application US2002/142457.

In certain embodiments of the present invention, differentiation is promoted by withdrawing one or more medium component(s) that promote(s) growth of undifferentiated cells, or act(s) as an inhibitor of differentiation. Examples of such components include certain growth factors, mitogens, leukocyte inhibitory factor (LIF), and basic fibroblast growth factor (bFGF). Differentiation may also be promoted by adding a medium component that promotes differentiation towards the desired cell lineage, or inhibits the growth of cells with undesired characteristics.

Thus, the method according to the present invention may further comprise the culturing of the composition in the presence of a differentiating agent and/or mitotic inhibitors, wherein a differentiation agent for inducing differentiation of the cell or cell line is selected from naturally occurring compounds and synthetic reagents such as retinoic acid, retinoids and derivatives thereof, bone morphogenic proteins, growth factors, trophic factors, macrophage inflammatory proteins, noggin, heparan sulfate, amphiregulin, interleukins and the like; see also Example 3. Differentiated cells and cell lines thereof are also subject of the present invention.

The tumor stem cells of the present invention are particularly suited for use in drug and toxicity screening as well as for therapeutic applications. For example, tumor stem cells of this invention can be used to screen for factors (such as solvents, small molecules, drugs, peptides, polynucleotides, and the like) or environmental conditions (such as culture conditions or manipulation) that affect the characteristics of the cells, for example during the process of differentiation. Particular screening applications of this invention relate to the testing of pharmaceutical compounds in drug research. It is referred generally to the standard textbook "In vitro Methods in Pharmaceutical Research", Academic Press, 1997, and US patent 5,030,015. Assessment of the activity of candidate pharmaceutical compounds generally involves combining the tumor stem cells or differentiated cells of this invention with the candidate compound, determining any change in the morphology, marker phenotype, or metabolic activity of the cells that is attributable to the compound (compared with untreated cells or cells treated with an inert compound), and then correlating the effect of the compound with the observed change. The screening may be done, for example, either because the compound is designed to have a pharmacological effect on certain cell types, or because a compound designed to have effects elsewhere may have unintended side effects. Two or more drugs can be tested in combination (by combining with the cells either simultaneously or sequentially) to detect possible drug-drug interaction effects. In some applications, compounds are screened initially for potential toxicity (Castell et al., pp. 375-410 in "In vitro Methods in Pharmaceutical Research," Academic Press, 1997). Cytotoxicity can be determined in the first instance by the effect on cell viability, survival, morphology, and expression or release of certain markers, receptors or enzymes. Effects of a drug on chromosomal DNA can be determined by measuring DNA synthesis or repair. [H]thymidine or BrdU incorporation, especially at unscheduled times in the cell cycle, or above the level required for cell replication, is consistent with a drug effect. Unwanted effects can also include unusual rates of sister chromatid exchange, determined by metaphase spread. It is referred to A. Vickers (pp 375-410 in "In vitro Methods in Pharmaceutical Research," Academic Press, 1997) for further elaboration.

Effects of cell function can be assessed using any standard assay to observe for example phenotypes or differentiability, extensive proliferative capacity, self-renewal and maintainance of the stem cell in the cell culture. Where an effect is observed, the concentration of the compound can be titrated to determine the median effective dose.

Effects of the test compound on cancer stem cells can also be measured for example by determining the number of cancer stem cells that persist in culture or in the tumors in vivo after treatment with the test compound. In addition to determining the number of cancer stem cells, the effects of the test compound on cancer stem cell cell-cycle status, and marker expression can also be determined by for example flow-cytometry. Furthermore, the effects of the test compounds or biological agents can be identified on the basis of significant differences relative to control cultures with respect to criteria such as the ratios of expressed phenotypes, cell viability, proliferation rate, number of cancer stem cells, cancer stem cell activity upon transplantation in vivo, cancer stem cell activity upon transplantation in culture, cell cycle distribution of tumor cells, and alterations in gene expression.

Resistance to chemotherapy is the main reason for the failure of current treatment of patients suffering from cancer. This resistance also termed "classical multidrug resistance" (MDR). Membrane transporters play important roles in mediating chemosensitivity and -resistance of tumor cells; see for review, e.g., Huang and Sadee, Cancer Lett. 2005 Sep 15, S0304-3835; Dean et al., Genome Res. 11 (2001), 1156-1166. ABC transporters, such as ABCB1/MDR1, ABCC1/MRP1 and ABCG2/BCRP, are frequently associated with decreased cellular accumulation of anticancer drugs and multidrug resistance of tumors. SLC transporters, such as folate, nucleoside, and amino acid transporters, commonly increase chemosensitivity by mediating the cellular uptake of hydrophilic drugs. Ion channels and pumps variably affect sensitivity to anticancer therapy by modulating viability of tumor cells.

Since it could be shown for YB-1 that oncogenes are involved in the development of multidrug resistance, alternatively, or in addition to the oncogene the overexpression or activity of the respective transporter proteins which actually cause the undesired efflux of drugs may be targeted; see Example 4.

Thus, in accordance with the present invention, the tumor stem cells can be used for investigating drug-transporter relationships and anticancer drug response; see Example 3. Hence, the present invention also relates to pharmacogenomic approaches, using correlations between drug potency and transporter gene expression in multiple cancer stem cell lines, for identifying potential drug-transporter relationships and predicting anticancer drug response, in an effort to optimize chemotherapy for individual patients. Any compounds such as drugs and blocking agents may be tested in accordance with the method of the present invention.

Such screening assays may of course be performed with normal stem cells in parallel or the results of one of such assay may be compared with a corresponding reference. In this context, expression profiling may be employed, for example of ABC transporters in breast cancer stem cells using array techniques such as described for a drug-resistant breast cancer cell line in Liu et al., Mol. Pharmacol. 68 (2005), 430-438. The relevance of the breast cancer resistance protein (ABCG2) in drug transport is discussed in Mao and Unadkat, AAPS J. 7 (2005), article 12, published online.

As already discussed hereinbefore, the provision of tumor stem cells enables the development of cell-based in vitro assays for screening compounds that specifically act on the tumor stem cell as well as for identifying novel targets for therapeutic intervention in the treatment of cancer. In principal, such screening assays are known in drug discovery and can be applied to the tumor stem cell of the present invention. For example, recent advances in cytotoxic anticancer drug assay design with emphasis on a novel mammalian cell-based anticancer drug finder technology for the discovery of cytotoxic drugs with fewer side-effects on non-dividing cells is provided by Gonzales-Nicolini and Fussenegger, Anticancer Drugs 16 (2005), 223-228. Furthermore, technological advances in high-throughput screening are summarized by Liu et al., Am. J. Pharmacogenomics 4 (2004), 263-276. The use of new approaches in identifying drugs to inactivate oncogene products including target validation with RNA interference and transgenic animal studies are described by Liu et al in Semin. Cancer Biol. 14 (2004), 13-21. Chemical approaches to the discovery and development of cancer therapies are discussed in Neidle and Thurston, Nat. Rev. Cancer 5 (2005), 285-296. With respect to micro array techniques, micro arrays of small molecules embedded in biodegradable polymers for use in mammalian cell-based screens is described in Bailey et al., Proc. Natl. Acad. Sci. USA 101 (2004), 16144-16149. In addition, automatic identification of subcellular phenotypes on human cell arrays including large-scale RNAi screening in human cells has been described in Conrad et al., Genome Res. 14 (2004), 1130-1136.

It will be appreciated that all these methods described in the references cited herein as well as others may be applied in accordance with the present invention. Accordingly, the following embodiments of the present invention can be easily performed in accordance with prior art methods. In particular, the screening methods disclosed in international application WO02/12447 can be adapted and applied in accordance with the present invention.
In a further embodiment the present invention relates to methods for obtaining and/or profiling a test substance capable of influencing stem cell development and differentiation, comprising the differentiation of stem cells and/or the steps of:
(a) contacting a test sample comprising said tumor stem cells or in vitro differentiated cells thereof with a test substance; and
(b) determining a phenotypic response in said test sample compared to a control sample, wherein a change in the phenotypic response in said test sample compared to the control sample is an indication that said test substance has an effect on cell development and/or tissue structure formation.
As mentioned, especially the connection of YB-1 with the development of a multidrug resistance is interesting, since here the major problem in the treatment of cancer can be investigated; see also Example 4.

In an addition, or alternative embodiment the present invention relates to a method for screening for a test compound that specifically binds to a tumor stem cell, comprising the steps of:
(a) contacting the tumor stem cell of the present invention or a population of such cells with a test compound under conditions suitable to allow complex formation; and
(b) detecting complex formation between the test compound and a tumor stem cell,
wherein the presence of the complex identifies the test compound as specifically binding the tumor stem cell.
For example, experiments for determining the binding of parathyroid hormone-related protein to heat shock protein 70 (HSP70) expressed on the surface of cancer cells has been described by Grzesiak et al., Endocrinology 146 (2005), 3567-3576. In this study, affinity chromatography applied on extracts of cell-surface biotinylated proteins from cancer and normal cell lines was used. Mass spectroscopy analysis and immunoblotting was used for identifying the cell surface protein of the cancer cell. Similarly, the tumor stem cells of the present invention can be analyzed compared to for example normal stem cells.
Accordingly, the method of the present invention may also further comprise the step of identifying the target in the contacted cells with which the test compound interacts.

The present invention also concerns exposure of the cells to radiation, for example in a radiation chamber, this radiation being for example electromagnetic radiation like radio waves, micro waves, infrared radiation, visible light, ultraviolet light, X-rays or γ-rays; particle radiation such as electron beam, neutron radiation, proton radiation and cosmic radiation, either alone or in combination.

Furthermore, the tumor stem cells of the present invention allow investigation whether a given compound is capable of preventing the onset of a disease like cancer. Therefore, the test compound may be solubilized and added to the culture medium at varying concentrations to determine the effect of the test compounds or agent at appropriate doses. The culture medium may be replenished with the test compound or biological agent at an appropriate time interval in amounts so as to keep the concentration of the agent somewhat constant. This embodiment is particularly useful for identifying and obtaining drugs that can be used as a prophylactic means, which is especially worthwhile considering for the prevention of diseases, especially cancer diseases. This prevention concerns for example "normal" stem cells to become carcinogenic, when treated with a preventing agent and then afterwards being exposed to carcinogenic agents like radiation, viruses or chemicals. Those carcinogenic agents include for example, but are not limited to radiation like UV light, ionizing radiation like X-rays, gamma rays, α-particles, β-particles, protons or neutrons; viruses like the human papilloma virus, hepatitis B, Epstein-Barr virus, human T-cell leukemia virus and Kaposi's sarcoma herpes virus; chemicals like benzene, organic solvents, arsenic, asbestos, vinyl chloride, volatilized chromium and nickel, as well as pollutants and tobacco products.

Monitoring the effects on proliferation or viability includes according to the invention monitoring the rate of cell proliferation or of cell progeny proliferation, monitoring the nature of cell differentiation and the ratio of differentiated cell types, monitoring symmetric and asymmetric division of (cancer) stem cells, monitoring cell death and the like, monitoring the changes in cell development or morphology, monitoring tumor formation and/or tumor growth, monitoring the changes in expressed phenotypes, amount or type of proteins expressed and the like, direction and ionic nature of current flow, dynamics of ion channels, monitoring changes in developmental characteristics like sensitivity towards and/or inducibility of apoptosis, adherence dependency or independency, controlled or uncontrolled cell growth, dependency or independency from other cell contacts, i.e. the "communication" between cells, monitoring up- and down regulation of enzymes and genes, especially growth factors and tumor suppressor genes, and monitoring "behavioral" characteristics like becoming autonomous and leaving an united cell structure.

For the screening methods of the present invention the tumor stem cells may be localized in monolayers in culture, in suspension in culture, or affixed to a solid surface. Preferably, the screening method is performed on an array; see also supra. Arrays for use in the assay of the present invention usually comprise a solid support and attached thereto or suspended thereon the stem cells or in vitro differentiated cells thereof.
Preferred compound formulations for testing do not include additional components, such as preservatives, that have a significant effect on the overall formulation; see also supra. Thus preferred formulations consist essentially of a biologically active compound and a physiologically acceptable carrier, e.g. water, ethanol, DMSO, etc. However, if a compound is liquid without an excipient the formulation may consist essentially of the compound itself.
Furthermore, a plurality of assays may be run in parallel with different compound concentrations to obtain a differential response to the various concentrations. As known in the art, determining the effective concentration of a compound typically uses a range of concentrations resulting from 1:10, or other log scale, dilutions. The concentrations may be further refined with a second series of dilutions, if necessary. Typically, one of these concentrations serves as a negative control, i.e. at zero concentration or below the level of detection.

Compounds and candidate agents are obtained from a wide variety of sources including libraries of synthetic or natural compounds. For example, numerous means are available for random and directed synthesis of a wide variety of organic compounds and biomolecules, including expression of randomized oligonucleotides and oligopeptides. Alternatively, libraries of natural compounds in the form of bacterial, fungal, plant and animal extracts are available or readily produced. For example, inhibition of tumor-induced angiogenesis and matrix-metalloproteinase expression in confrontation cultures of embryoid bodies and tumor spheroids by plant ingredients used in traditional Chinese medicine has been described by Wartenberg et al. in Lab. Invest. 83 (2003), 87-98.

The test compound may optionally be a combinatorial library for screening a plurality of compounds. Such a collection of test substances can have a diversity of about 10³ to about 10⁵ is successively reduced in running the method, optionally combined with others twice or more. Compounds identified by the method of the invention can be further evaluated, detected, cloned, sequenced, and the like, either in solution or after binding to a solid support, by any method usually applied to the detection of a specific DNA sequence such as PCR, oligomer restriction (Saiki et al., Bio/Technology 3 (1985), 1008-1012, allele-specific oligonucleotide (ASO) probe analysis (Conner et al., Proc. Natl. Acad. Sci. USA 80 (1983), 278), oligonucleotide ligation assays (OLAs) (Landegren et al., Science 241 (1988), 1077), and the like. Molecular techniques for DNA analysis have been reviewed (Landegren et al., Science 242 (1988), 229-237). Hence, the method of the present invention can also be used for transcriptional profiling of tumor stem cells and in vitro differentiated cells thereof; see, e.g., Ramalho-Santos et al., Science 298 (2002), 597-600; Tanaka et al., Genome Res. 12 (2002), 1921-1928.

The tumor cell-based assay of the present invention is particularly suited to provide modulation reference patterns, i.e. profiles and databases of modulation reference patterns for a wide range of biologically active compounds. The reference patterns are then used for the identification and classification of test compounds. Evaluation of test compounds may be used to achieve different results.

In one embodiment of the present invention, the above described methods further comprise characterization of tumor stem cells or in vitro differentiated cells thereof including the measurement of for example changes in transcription activity, transcription level, translation activity, translation level, protein expression, protein levels, quantity of for example nucleic acids or proteins, processing of nucleic acids (DNA and/or RNA), changes in protein function or activity, alteration in tissue composition or function, changes in gene expression or gene expression levels (especially tumor suppressor genes), changes in activity, intactness and/or functionality as well as altered quantity of factors such as for example co-factors, transcription factors or growth factors, changes in binding affinities of factors, proteins, enzymes or nucleic acid molecules, up- and/or down-regulation of nucleic acids and/or proteins; changes in metabolism, gain or loss of polymorphisms, altered response to external stimuli or changes in sequence compositions of nucleic acids and/or proteins, which are significantly different from "normal" stem cells. Those changes can be measured using means and methods known to those skilled in the art.

US patent. No. 6,194,158 refers to a diagnostic assay for cancer with a DNA chip of specific sequences for measuring expression levels of certain sequences within a cancer cell to determine whether expression is up- or down-regulated. The DNA chip comprising nucleotide sequences capable of hybridizing to one or more members of a panel of DNA sequences may be synthesized using commonly available techniques. mRNA is isolated from a normal, non-cancer cell and a cancer cell and hybridized to the DNA chip comprising one of more of the sequences from the panel. Hybridization is then detected by any of the available methods known to those skilled in the art. In such a manner, sequences that are either overexpressed or underexpressed in a cancer cell as compared to a normal cell are detected. In a similar manner, mRNA from a cancer cell that has been contacted with a compound may be hybridized to sequences on the DNA chip to determine whether that compound affects expression of a particular sequence. The present invention provides an improvement over this method, in that the "cancer cell" from which mRNA can be isolated is the cancer stem cell of the present invention.

For example, once a differentially expressed gene has been identified and isolated, therapeutic agents can be directed against the gene or gene product in order to specifically target and eliminate the tumorigenic subset of tumor stem cells within the tumor that have the capacity for extensive proliferation and the ability to give rise to all other tumor cell types.

In a particularly preferred embodiment, any one of the above-described methods of the present invention comprises the use of a microarray; see also supra. Since most microarray assays are nucleic based assays is of course understood that the methods of the present invention include the isolation of nucleic acids, preferably mRNA from the tumor stem cells or in vitro differentiated cells and, if necessary, their further processing for making them suitable for the array analysis. Hence, microarrays are particular useful for gene expression profiling of tumor stem cells and include for example Affymetrix GeneChip^{©} microarrays; see for example the recent publication by Maitra et al., Nature Genetics 37 (2005), 1099-1103, in which the characterization of stem cell lines using such microarrays is described. Moreover, current microarray, serial analysis of gene expression (SAGE), and expressed sequence tags (EST) strategies to characterize stem cells and their differentiated derivatives are summarized in Robson, Trends Biotechnol. 22 (2004), 690-612. Gene expression profiling of embryonic stem cells is further described by Rao and Stice in Biology of Reproduction 71 (2004), 1772-1778. In addition, studies of stem cell function using microarray experiments have been performed by Perez-Iratxeta et al. and described in FEBS Lett. 579 (2005), 1795-1801. In this publication, the authors provide a collection of mRNA measurements in different cell types and states and use the results as a source of functional predictions. The data can be accessed online via the internet reference given.
Hence, corresponding expression profiles can be generated for the tumor stem cells of the present invention and compared to those published and available for "normal" stem cells, thereby identifying putative therapeutic cancer targets.

As mentioned, the assay methods of the present invention can be in conventional laboratory format or adapted for high throughput screening. The term "high throughput" (HTS) refers to an assay design that allows easy analysis of multiple samples simultaneously, and capacity for robotic manipulation. Another desired feature of high throughput assays is an assay design that is optimized to reduce reagent usage, or minimize the number of manipulations in order to achieve the analysis desired. In this context microfluidic methods may be used as well as screening platforms, wherein the test compound or the stem cells are attached to a solid surface.

Examples of conventional assay formats include 96-well, 384-well or more-well plates, levitating droplets, and "lab on a chip" microchannel chips used for liquid handling experiments. It is well known by those skilled in the art that as miniaturization of plastic molds and liquid handling devices are advanced, or as improved assay devices are designed, that greater numbers of samples may be performed using the design of the present invention. Two or more measurements, optionally at different positions within the array may be taken. Several test substances can be combined and either added simultaneously or sequentially to gain information about possible enhancing or quenching effects. Thus a further aspect of the invention relates to the method described previously, wherein the contacting step further includes contacting the test sample with at least one second test substance in the presence of said first test substance. Two or more substances tested in combination will provide information about their interaction in general and optionally a compound known to activate or inhibit a disease process may be added to the sample or culture medium.

Furthermore, the above-described methods can, of course, be combined with one or more steps of any of the herein described screening methods or other screening methods well known in the art. Methods for clinical compound discovery comprises for example ultrahigh- throughput screening (Sundberg, Curr. Opin. Biotechnol. 11 (2000), 47-53) for lead identification, and structure-based drug design (Verlinde and Hol, Structure 2 (1994), 577-587) and combinatorial chemistry (Salemme et al., Structure 15 (1997), 319-324) for lead optimization. Once a drug has been selected, the method can have the additional step of repeating the method used to perform rational drug design using the modified drug and to assess whether said modified drug displays better affinity according to for example interaction/energy analysis. The method of the present invention may be repeated one or more times such that the diversity of said collection of compounds is successively reduced.

Furthermore, the present invention relates to the therapeutic agent, i.e. anti-cancer drug identified or isolated according to the screening methods of the present invention described hereinbefore, which agent hitherto has not been known or used as an anti-cancer drug. Hence, the present invention also relates to compositions comprising said agent for use in therapy and diagnosis of cancer as well as in diagnostic assays. The drug according to the invention can be combined with suitable diluents or carriers, preferably those which are pharmaceutically acceptable. Examples of such carriers, diluents and methods of formulation may be found in Remington's Pharmaceutical Sciences, 20th ed. (Mack Publishing Co., Easton, PA). To form a pharmaceutically acceptable composition suitable for effective administration, such compositions will contain an effective amount of the drug. Carriers or diluents are usually sterile and non-toxic, and defined as vehicles commonly used to formulate pharmaceutical compositions for animal or human administration. The diluent is selected so as not to affect the biological activity of the combination. Examples of such diluents are distilled water, physiological saline, Ringer's solutions, dextrose solution, and Hank's solution. In addition, the pharmaceutical composition or formulation may also include other carriers, adjuvants, or non-toxic, non-therapeutic, non-immunogenic stabilizers and the like. Further examples of suitable pharmaceutical carriers are well known in the art and include phosphate buffered saline solutions, emulsions, such as oil/water emulsions, various types of wetting agents, sterile solutions etc. Compositions comprising such carriers can be formulated by well known conventional methods; see Remington's Pharmaceutical Sciences, supra.

Once a drug has been selected in accordance with any one of the above-described methods of the present invention, the drug or a pro-drug thereof can be synthesized in a therapeutically effective amount. As used herein, the term "a therapeutically effective amount" means the total amount of the drug or pro-drug that is sufficient to show a meaningful patient benefit, i.e. treatment, healing, prevention or amelioration of damaged tissue, or an increase in the rate of treatment, healing, prevention or amelioration of such conditions. In addition or alternatively, in particular with respect to pre-clinical testing of the drug the term "therapeutically effective amount" includes the total amount of the drug or pro-drug that is sufficient to elicit a physiological response in a non-human animal test.

The appropriate concentration of the therapeutic agent might be dependent on the particular agent. The therapeutically effective dose has to be compared with the toxic concentrations; the clearance rate; as well as the metabolic products play a role as to the solubility and the formulation. Therapeutic efficacy and toxicity of compounds can be determined by standard pharmaceutical procedures in cell cultures or experimental animals, e.g., ED50 (the dose therapeutically effective in 50 % of the population) and LD50 (the dose lethal to 50 % of the population). The dose ratio between therapeutic and toxic effects is the therapeutic index, and it can be expressed as the ratio, LD50/ED50.

Depending on the specific cancer being treated, agents may be formulated and administered systemically or locally. Techniques for formulation and administration may be found in Remington's Pharmaceutical Sciences, supra. Other suitable routes may include oral, rectal, transdermal, vaginal, transmucosal, or intestinal administration; parenteral delivery, including intramuscular, subcutaneous, intramedullary injections, as well as intrathecal, direct intraventricular, intravenous, intraperitoneal, intranasal, or intraocular injections, just to name a few.

Thus, the present invention relates to the use of a compound identified, isolated and/or produced by any one of the methods of the present invention for the preparation of a composition for the treatment of aberrant cells, tissue or organ formation, in particular cancer; see also supra. Preferably, the isolated compound or corresponding drugs support prevention of tumor formation and/or tumor growth, selective removal of cancer stem cells to prevent recurrence of cancer, and/or making cancer cells accessible to chemotherapeutic agents.

The tumor stem cells of the present invention can of course also be used for further investigation in vivo. For example, isolated tumor stem cells can be grafted into immunocompromised mice and analyzed for their capacity to form new tumors. Prior to introducing the purified tumor stem cells into the animal they may of course be subjected to for example treatment with putative anti-tumor agents or gamma-irradiation. Furthermore, they may be genetically engineered, for example in order to assess the therapeutic effect of a putative tumor suppressor gene. A corresponding animal xenograft model in which for example human breast cancer stem cells may be grown in immunocompromised mice is described in international application WO03/050502, the disclosure content of which is incorporated herein by reference.
International application WO03/050502 also provides information and references concerning animal xenograft models in general, for example with respect to a laboratory mammal such as a mouse, e.g. nude mouse, SCID mouse, NOD/SCID mouse as well as rat, rabbit and primate. Furthermore, general techniques for formulation and injection of cells into the animal are described, which however can also be found in general textbooks such as Remington's Pharmaceutical Sciences, 20th ed. (Mack Publishing Co., Easton, PA).
Thus, in a further embodiment the present invention relates to a method for determining the effect of a test compound on a tumor stem cell, comprising the steps of:
(a) transplanting the tumor stem cell of the present invention or a population of such cells into an immunocompromised mouse;
(b) administering a test compound to the immunocompromised mouse; and
(c) determining the response of the transplanted tumor stem cell to the test compound.
As described previously, mice can be further immunosuppressed by a method selected from the group consisting of administration of VP16, radiation therapy and chemotherapy. Furthermore, the means and methods described in international application WO03/089580 for using stem cells to make chimeric non-human animals having cancer or the ability to develop cancer may be employed and adapted in accordance with the teaching of the present invention.

The present invention also relates to one or more kits for using the tumor stem cells of the present invention, said kits containing specific reagents such as those described hereinbefore useful for conducting any of the above-described methods of the present invention, comprising culture medium or culture media components, growth factors, selectable markers, reference samples, microarrays, chelating substances, culture vessels, cell suspending media, blocking media, vectors, nucleic acid probes, or the like. Such a kit would typically comprise a compartmentalized carrier suitable to hold in close confinement at least one container and the compounds of the kit may be sterile, where appropriate. The kit may further include a transfer means, such as pipets, for transferring the suspended cells.
The kit's carrier could further comprise reagents useful for performing said methods and may also contain means for detection such as labeled enzyme substrates or the like. Instructions can be provided to detail the use of the components of the kit, such as written instructions, video presentations, or instructions in a format that can be opened on a computer (e.g. a diskette or CD-ROM disk). These instructions indicate, for example, how to use the cells to screen test agents of interest.

As already discussed in context with the assay system of the present invention for screening putative drugs, the observations made in accordance with the present invention can also be applied to establish a novel method of identifying putative target genes for therapeutic intervention within the treatment of cancer. Instead of developing the identified drug in house, further drug development can also be achieved by a different company. Thus, a further aspect of the present invention relates to a method of conducting a target discovery tumor stem cell technology business comprising (a) the provision of information on the testing of compounds in accordance with the method of compound screening as described supra; (optionally) conducting therapeutic profiling of anti-tumor agents identified in step (a) for efficacy and toxicity in animals; and (c) licensing, to a third party, the rights for further drug development and/or sales for anti-tumor agents identified in step (a) or (b), or analogs thereof.
For suitable lead compounds that have been identified further profiling of the agent, or further analogs thereof, can be carried out for assessing efficacy and toxicity in animals, depending on the modalities of the agreement with the respective third party. Further development of those compounds for use in humans or for veterinary uses will then be conducted by the third party. The subject business method will usually involve either the sale or licensing of the rights to develop said compound but may also be conducted as a service, offered to drug developing companies for a fee.

In addition, the present invention relates to a tumor stem cell bank, comprising tumor stem cells of the present invention, preferably wherein said cells are capable of responding to external agents such as drugs and pharmaceuticals for screening.

Hence, the present invention further relates to the use of tumor stem cells or differentiated cells thereof, as described hereinbefore in a variety of applications including, but not limited to "loss of function" assays of specific genes, "gain of function" assays of exogenous genes, analysis and screening of putative drugs, pharmacological assays, microarray systems, establishment of model systems for pathological cell functions, testing of anti-tumor agents, for investigating differentiation in vitro and/or in vivo, for compound testing or screening in pharmaceutical development.

In one embodiment of the invention further characterization of cancer stem cells comprises the measurement of for example changes in transcription activity, transcription level, translation activity, translation level, protein expression, protein levels, quantity of for example nucleic acids or proteins, processing of nucleic acids (DNA and/or RNA), changes in protein function or activity, alteration in tissue composition or function, changes in gene expression or gene expression levels (especially tumor suppressor genes), changes in activity, intactness and/or functionality as well as altered quantity of factors such as for example co-factors, transcription factors or growth factors, changes in binding affinities of factors, proteins, enzymes or nucleic acid molecules, up- and/or down-regulation of nucleic acids and/or proteins; changes in metabolism, gain or loss of polymorphisms, altered response to external stimuli or changes in sequence compositions of nucleic acids and/or proteins, which are significantly different from "normal" stem cells. Those changes can be measured using means and methods known to those skilled in the art.

The microarray as described hereinbefore may further be used to monitor the progression of tumor development by assessing and cataloging the differences in gene expression of tumor stem cells during tumorigenesis based on the analysis of the changes in patterns of gene expression compared to the initial tumor stem cell and normal stem cells, respectively. The present invention can also be used to monitor the efficacy of treatment. For some treatments with known side effects, the microarray is employed to "fine tune" the treatment regimen. A dosage is established that causes a change in genetic expression patterns indicative of successful treatment. Expression patterns associated with undesirable side effects can be avoided. This approach may be more sensitive and rapid than waiting for the patient to show inadequate improvement, or to manifest side effects, before altering the course of treatment.

Alternatively, the "stem cell" qualities of the cancer stem cells of the invention, protein extracts derived from said cells, purified proteins from said cancer stem cells, or proteins derived from the expression of cDNAs from said cancer stem cells (see also genetic modification of cancer stem cells, as described) may be used to induce an immune response in an animal for example. The immune response can be directed against cancer stem cells, as shown by standard immunological methods. For example, the tumor stem cells of the present invention or surface proteins and extracellular exposed peptides thereof can be contacted with dendritic cells in culture, antigen presenting cells in culture, or antigen presenting cells and T cells in culture. Then antigen-stimulated cells may be infused back into the patient. Thus, the tumor stem cell the present invention may further be used for the vaccination of animals to get monoclonal antibodies or at least reactive cells.

The disclosure content of international application WO02/12447 has already been discussed before. Though not relevant prior art to the present invention, this international application nevertheless provides useful information on for example xenograft models of human breast cancer, see paragraph [51], genetic modification of stem cells, see paragraph [94], as well as genetic manipulation techniques and methods for expression analyses in general. All these means and methods described in the section "Modes for carrying out the invention" and partially illustrated in the examples, as well as the cited references are incorporated in the disclosure of the present invention and are not repeated in full for the sake of conciseness. International application WO03/066840 describes further applications of stem cells, in particular pluripotent embryonic-like stem cells, preferably derived from teeth.

In view of the contribution of the present disclosure to cancer research, it will be acknowledged that the present invention also relates to the use of any tumor promoting agent such as those described hereinbefore and the nucleic acid molecules encoding such tumor promoting agents for inducing a tumor in a non-human animal as a source for tumor stem cells. Likewise, the present invention relates to the use of a non-human animal comprising or determined to develop a tumor due to the presence of a foreign nucleic acid molecule encoding a tumor promoting agent as a source for the isolation of tumor stem cells from said tumor. Preferably, said tumor promoting agent is a human oncogene or a tumor promoting fragment thereof, most preferably said oncogene is the YB-1 gene.

It will be apparent that the tumor stem cells, methods and uses as substantially described herein or illustrated in the description and the examples are also subject of the present invention and claimed herewith. In this respect, it is also understood that the embodiments as described in any one of the examples can be independently used and combined with any one of the embodiments described hereinbefore and claimed in the appended claims set.

These and other embodiments are disclosed and encompassed by the description and examples of the present invention. Further literature concerning any one of the materials, methods, uses and compounds to be employed in accordance with the present invention may be retrieved from public libraries and databases, using for example electronic devices. For example the public database "Medline" may be utilized, which is hosted by the National Center for Biotechnology Information and/or the National Library of Medicine at the National Institutes of Health. Further databases and web addresses, such as those of the European Bioinformatics Institute (EBI), which is part of the European Molecular Biology Laboratory (EMBL) are known to the person skilled in the art and can also be obtained using internet search engines. An overview of patent information in biotechnology and a survey of relevant sources of patent information useful for retrospective searching and for current awareness is given in Berks, TIBTECH 12 (1994), 352-364.

The above disclosure generally describes the present invention. Several documents are cited throughout the text of this specification. Full bibliographic citations may be found at the end of the specification immediately preceding the claims. The contents of all cited references (including literature references, issued patents, published patent applications as cited throughout this application and manufacturer's specifications, instructions, etc) are hereby expressly incorporated by reference; however, there is no admission that any document cited is indeed prior art as to the present invention.

The above disclosure generally describes the present invention. A more complete understanding can be obtained by reference to the following specific examples which are provided herein for purposes of illustration only and are not intended to limit the scope of the invention.

### EXAMPLES

The examples which follow further illustrate the invention, but should not be construed to limit the scope of the invention in any way. Detailed descriptions of conventional methods, such as those employed herein can be found in the cited literature; see also "The Merck Manual of Diagnosis and Therapy" Seventeenth Ed. ed by Beers and Berkow (Merck & Co., Inc. 2003).

The practice of the present invention will employ, unless otherwise indicated, conventional techniques of cell biology, cell culture, molecular biology, transgenic biology, microbiology, recombinant DNA, and immunology, which are within the skill of the art.
Methods in molecular genetics and genetic engineering are described generally in the current editions of Molecular Cloning: A Laboratory Manual, (Sambrook et al., (1989) Molecular Cloning: A Laboratory Manual, 2nd ed., Cold Spring Harbor Laboratory Press); DNA Cloning, Volumes I and II (Glover ed., 1985); Oligonucleotide Synthesis (Gait ed., 1984); Nucleic Acid Hybridization (Hames and Higgins eds. 1984); Transcription And Translation (Hames and Higgins eds. 1984); Culture Of Animal Cells (Freshney and Alan, Liss, Inc., 1987); Gene Transfer Vectors for Mammalian Cells (Miller and Calos, eds.); Current Protocols in Molecular Biology and Short Protocols in Molecular Biology, 3rd Edition (Ausubel et al., eds.); and Recombinant DNA Methodology (Wu, ed., Academic Press). Gene Transfer Vectors For Mammalian Cells (Miller and Calos, eds., 1987, Cold Spring Harbor Laboratory); Methods In Enzymology, Vols. 154 and 155 (Wu et al., eds.); Immobilized Cells And Enzymes (IRL Press, 1986); Perbal, A Practical Guide To Molecular Cloning (1984); the treatise, Methods In Enzymology (Academic Press, Inc., N.Y.); Immunochemical Methods In Cell And Molecular Biology (Mayer and Walker, eds., Academic Press, London, 1987); Handbook Of Experimental Immunology, Volumes I-IV (Weir and Blackwell, eds., 1986). Reagents, cloning vectors, and kits for genetic manipulation referred to in this disclosure are available from commercial vendors such as BioRad, Stratagene, Invitrogen, and Clontech. General techniques in cell culture and media collection are outlined in Large Scale Mammalian Cell Culture (Hu et al., Curr. Opin. Biotechnol. 8 (1997), 148); Serum-free Media (Kitano, Biotechnology 17 (1991), 73); Large Scale Mammalian Cell Culture (Curr. Opin. Biotechnol. 2 (1991), 375); and Suspension Culture of Mammalian Cells (Birch et al., Bioprocess Technol. 19 (1990), 251); Extracting information from cDNA arrays, Herzel et al., CHAOS 11, (2001), 98-107.

### Example 1: Generation of designer tumor mice for isolating cancer stem cells

As mentioned in the description hereinbefore, transgenic animals expressing human YB-1 and as a consequence of overexpressing of said transgene (YB-1) developing human tumors are used, such as described in Bergmann et al., Cancer Res. 65 (2005), 4078-4087. Briefly, for the heterologous expression of YB-1 preferably the β-lactoglobulin (BLG) promoter is used, like for example in expression vector pBJ41 containing the 4.3 kilo bases of the 5' flanking sequence of the BLG gene, its exons 1, 7 and 8, and 1.9 kb of the BLG gene's 3' flanking sequence. Plasmid pBJ41 is an expression vector which harbors an ovine β-lactoglobulin (BLG) promoter suitable to direct expression of a foreign gene during pregnancy and lactation; see Lundgren et al., Genes Dev. 11 (1997), 714-725. The complete nucleotide sequence of the genomic BLG gene including indication of the promoter region is described in Harris et al., Nucleic Acids Res. 16 (1988), 10379-10380 and Watson et al., Nucleic Acids Res. 19 (1991), 6603-6610. In particular reference is made to GenBank Accession Number X12817 [gi:1313].

The construction of expression cassette pBJ41 is described by Sola et al., Virol. 72 (1998), 3762-3772. In this publication reference is made to another appropriate BLG construct pSS1tgXS that has been described previously, see, e.g., Ali and Clark, J. Mol. Biol. 199 (1988), 415-426; Harris et al., Dev. Genet. 12 (1991), 299-307; Simons et al., Nature 328 (1987), 530-532, and comprises 4.3 kb of 5'-flanking sequences, the 4.9-kb transcription unit, and 1.7 kb of 3'-flanking sequences of the B allele of sheep BLG; see Ali et al., Gene 91 (1990), 201-207. The BLG promoter has also been used for the induction of mammary gland hyperplasia and carcinomas in transgenic mice expressing human cyclin; see Bortner and Rosenberg, Mol. Cell. Biol. 17 (1997), 453-459. The above cited references also describe methods for the generation of transgenic mice, which may thus also be applied with respect to the present invention.

As mentioned in the description, the nucleotide sequence of the YB-1 cDNA is deposited with Accession Numbers NM_004559 [gi:34098945] exemplified in SEQ ID NO: 1. The YB-1 cDNA is preferably fused to a sequence encoding a 9 to 12-residue carboxy-terminal influenza A virus hemagglutinin (HA) epitope tag. The sequence of the HA tag may differ from the common HA epitope (YPYDVPDYA; SEQ ID NO: 3), for example (YDVPDYASL; SEQ ID NO: 4) but still correspond to 9 residues of the 12-residue peptide used to raise commercially available anti-HA antibodies (Covance Research Products Inc., Richmond, Calif.). The YDVPDYASL tag is also recognized by the commercially available anti-HA antibodies.
Preferably, an EcoRI/EcoRI fragment of the human YB-1 cDNA is fused to the HA tag. The HA-tagged YB-1 cDNA can be cloned into pBJ41, using an EcoRV site which may be generated from a PvuII site in the untranslated region of exon 1. YB-1/HA can be obtained by PCR amplification of the HA sequence, wherein an EcoRI site at the 5'end of the HA sequence is generated. The BLG-YB-1/HA construct may then be obtained by subcloning the 1.4 kb YB-1/HA fragment into the EcoRV site of pBJ4l. Digestion with Xho/XbaI releases the 7.9 kb BLG-YB-1/HA fragment from the vector and subsequently this DNA fragment can be separated from the vector by electrophoresis using for example a 1 % low-melt agarose gel in 1x Tris-borate EDTA. After the gel slices containing BLG-YB-1/HA DNA being digested with for example QiaEX (Qiagen, Hilden, Germany) according to the manufacturer's recommendations, the DNA can be suspended in microinjection buffer (10 mmol/l Tris-HCl, 0.1 mmol/l EDTA (pH 7.4) to a final concentration of 10 ng/µl. The following microinjection of NMRI mouse embryos can be carried out as described by Brand (Brand et al., Oncogene 19 (2000) 2129-2137).

Subsequent to the microinjection, the DNA can be isolated from the tail of the animal (1 cm biopsies, Brand et al., Oncogene 19 (2000) 2129-2137), and the transgenic founders can be identified by PCR analysis. For PCR analysis any primer can be used, leading to the specific amplification of the target gene, i.e. the injected BLG-YB-1/HA construct. As known to those in the art it is suggestive to use sense and antisense primers, respectively specific for HA and YB-1, said primer sequences being for example 5'-GCG GCC GCT TCA AGC GTA ATC TGG AAC GTC-3' (SEQ ID NO: 5) for an HA-specific primer and 5'-ACC ATG GAG GGA TCG GAG AGT GCT CCC-3' (SEQ ID NO: 6) for the carboxy-terminal of YB-1, respectively (Bergmann et al., Cancer Res. 65 (2005), 4078-4087). Using this primer set it is possible to detect a 0.4 kb product in BLG-YB-1/HA transgenic (tg) founder lines, if PCR is carried out according to standard conditions: 30 seconds at 94 °C (denaturing), followed by 36 cycles for 30 seconds at 94 °C, 30 seconds at 55 °C, and 1 minute at 72 °C (amplification) and finally 10 minutes at 72 °C in a volume of 50 µl. 10 µl of the resulting PCR product can be analyzed by size fractionating in a 1 % agarose gel. As size standards, a plurality of molecular weight markers may be used, known to those skilled in the art, allowing the size determination of the PCR products, those markers being commercially available at for example Sigma, Roche, Amersham, just to name a few. The expression of the transgene can be examined by reverse transcription PCR (RT-PCR) analysis. Therefor RNA can be prepared as for example described in Chomczynski and Sacchi, Anal. Biochem. 162 (1987), 156-159. The amount of total RNA which is applied to RT-PCR may be for example 25 µg, but can vary within a certain range, depending on several factors, like for example activity of the enzyme, time of reaction and so forth, known to those skilled in the art. A plurality of reverse transcriptases (for RT) as well as DNA polymerases (for PCR) can be used, those enzymes being commercially available at for example Sigma, Roche, Invitrogen, and are known to the skilled person. The PCR that follows RT can be carried out under the conditions as described hereinbefore.

Histopathological analysis of transgenic mice and immunohistology, whole mount preparation of mammary glands, preparation of protein extracts and immunoblot analysis, etc. are described in detail in Bergmann et al., Cancer Res. 65 (2005), 4078-4087.

### Example 2: Isolation, enrichment and cultivation of cancer stem cells derived from YB-1 transgenic tumors

Tumors of two different YB-1 transgenic mice were separately treated as described in the following: Tumors were dissected into small pieces using a scalpel and enzymatically digested by a mixture of collagenase/dispase (Roche) into single cells. The thereby obtained cells further were seeded into Dulbecco's Modified Eagle's Medium (DMEM) low Glucose/Roswell Park Memorial Institute) Media (RPMI) (1:1) containing stable glutamine (Biochrom) and 10 % fetal bovine serum (FBS). The results thereof were cell lines with a heterogenic but predominantly fibroblastoidal morphology containing only few suspension cells, as shown in Fig. 1, wherein a) and b) refer to those obtained from mouse number one and mouse number two, respectively. The big adherent cells almost showed no proliferative activity. In case of removing the flexible suspension cells, for example by washing procedures, the proliferative activity of the whole culture declines, which was shown in longer time intervals till the next passage. This demonstrates that the higher proliferative activity is with the suspension cells and that these cells maintain the culture.

Cancer stem cells were enriched by centrifugation the culture medium during medium change at a rotation speed of 1000 g for 15 minutes, discarding the supernatant, resuspending the pellet (centrifuged material) in fresh medium and giving it back into the culture flasks. In doing so, small cancer stem cells could be enriched, which partially grew also in spherical forms, as exemplarily shown for mouse one in Fig. 2.

Besides the already described medium, DMEM containing 20 % serum replacement (Invitrogen) and different cytokines was used as well. Basic fibroblast growth factor (bFGF), epidermal growth factor (EGF) and a mixture of bFGF, EGF, bone morphogenetic protein (BMP-4) and platelet-derived growth factor (PDGF-BB) was tested in each case with and without leukemia inhibitory factor (LIF). Thereby, it turned out that the combination of bFGF (10 ng/ml) and LIF (1000 U/ml) worked best concerning optimal growth and substantially negligible differentiation. The cell cultures derived therefrom also showed spheroidal growth (Fig. 3, mouse one), but also single small cancer stem cells in suspension or adhered to cells. Even the culture containing EGF and the cell mixture also showed similar results so that further experiments were focused on bFGF with LIF as medium supplement.

### Example 3: Induced in vitro differentiation of cancer stem cells

A mixture of prolactin (1 µg/ml), dexamethasone (10⁻⁷ M) and insulin (10 µg/ml) (PDI) was added to the cultures of tumor stem cells derived from mouse one and mouse two, respectively, in DMEM/RPMI (1:1) containing 10 % FBS for the purpose of induced differentiation in lactating cells. Fig. 4 shows one of the cultures (mouse two) 18 days after induction at a magnification of 630. Surprisingly, there was a massive increase in sphere formation and formation of small suspension cells, also detectable in Fig. 5a, at a magnification of 100. One month after induction, the mass of spheres and suspension cells had further increased (see Fig. 5b). Additionally, now also cells with a strong fat production were detectable. Fig. 6 shows a culture of mouse two at a magnification of 100, wherein one area with an accumulation of fat-producing cells is detectable. In general, the cells differentiate only little, but rather more spheres and suspension cells developed, which could have been caused by the simultaneously induced YB-1 expression. Fig. 7 shows a differentiated fat-producing cell at high magnification (mouse one, magnification of 630). Thus, it was proven that stem cells are present with the cultivated cells, since differentiability is one major characteristic of stem cells.

### Example 4: Pharmaceutical characterization of the cancer stem cells

Cells derived from the "normal" culture (DMEM/RPMI, 10 % FBS), being breeded in a 75 cm² cell culture flask up to a confluence of 85 % were washed with PBS. Adhering cells were treated with Hank's Buffered Salt Solution (HBSS)/EDTA for 15 minutes to detach them. The thus harvested cells were transferred into DMEM containing 2 % FBS and treated as follows, each in the same basic medium:
1) Blocking with 50 µM verapamil (affecting P-glycoprotein and ABCB1), 10 µM fumitremorgin C (affecting ABCG2, *MXR*/*BCRP*/*ABCP*). The control was unblocked. Incubation was carried out at 37 °C for 60 minutes.
2) Addition of Hoechst33342, (5 µg/ml) and antibody: AC133 PE conjugate (Milteny). Incubation was carried out at 37 °C for 90 minutes.
3) Washing three times with PBS, followed by centrifugation for 15 minutes at 1800 rpm.
4) Sieving of the cells through a cell sieve having a mesh size of 40 µm
5) Fluorescence-Activated Cell Sorter (FACS) analysis

Recent research has demonstrated that stem cells in a variety of tissues, including bone marrow, skeletal muscle and fetal liver can be identified by their ability to efflux fluorescent dyes such as for example Hoechst 33342. Such a population is called the "side population" or SP, based on its profile on the flow cytometer. The following tables (table 1 and 2) show the analysis results of the cells which migrate from gate P1 to gate P2 during blocking. In the bone marrow of mice 0.58 % side population (SP) cells were found using fumitremorgin C (Telford and Frolova, Cytometry A. 57 (2004), 45-52). In most literature references, the portion of SP cells which can be blocked by verapamil is indicated as amounting to about 0.4 %. This showed even in the "normal" cultures a high amount of tumor cells in mouse one and two, which were able to protect themselves by an active efflux mediated by P-glycoprotein or BCRP.

**Table 1:**

| mouse 1 | control | verapamil | fumitremorgin C |
|---|---|---|---|
| P1 % | 76.1 | 74 | 61,4 |
| P2 % | 23.9 | 26 | 38.6 |
| SP % | | 2.1 | 14.7 |

**Table 2:**

| mouse 2 | control | verapamil | fumitremorgin C |
|---|---|---|---|
| P1 % | 70.8 | 66.8 | 62.9 |
| P2 % | 29.2 | 33.2 | 37.1 |
| SP % | | 4 | 7.9 |

Furthermore, a clear correlation of especially high CD133 expression to a very low Hoechst signal was found, further indicating the existence of stem cells, since CD133 represents a marker of early stem cells.

Hence, this is the first time it could be shown that tumors, in particular tumors of a non-human animal induced by the presence of a human oncogene, contain stem cells which can be cultivated and differentiated like native pluripotent stem cells. Thus, it is possible to isolate cancer stem cells from transgenic animals and make them accessible for further investigation. In particular, cancer stem cells have been isolated out of the tumors of transgenic animals and successfully established in culture, their stem cell properties have been shown as well as their potency of being directed to differentiate in vitro into a predetermined cell type; see Examples 2 and 3. In accordance with the present invention, this corroborates cancer stem cell theory and opens up new diagnostic and therapeutic possibilities. Additionally, the investigation of those tumor stem cells enables to gain insights into the formation of a tumor.

## Claims

1. A tumor stem cell which is derived from a tumor of a non-human animal, wherein said tumor is induced by the presence of or exposure to a tumor inducing agent.

2. The tumor cell of claim 1, wherein said tumor inducing agent is a foreign nucleic acid molecule, which is preferably derived from a tumor promoting gene.

3. A method for enriching tumor stem cells in a population of cells, comprising the steps of:
(a) dissociating a tumor as defined in claim 1 or 2 to form a cell suspension;
(b) culturing said cell suspension under conditions appropriate for maintenance and/or proliferation of stem cells, optionally
wherein the tumor stem cell selection is performed by change of medium, washing step, centrifugation, flow cytometry, fluorescence activated cell sorting, panning, affinity column separation, and/or magnetic selection.

4. A method for analyzing the tumor stem cell of claim 1 or 2, comprising analyzing said tumor stem cell for gene expression patterns.

5. A method for determining the effect of a test compound on a tumor stem cell, comprising the steps of:
(a) contacting the tumor stem cell of claim 1 or 2 with the test compound; and
(b) determining the response of the contacted cells to the test compound, optionally wherein the tumor stem cells are localized in a manner selected from the group consisting of in monolayers in culture, in suspension in culture, and affixed to a solid surface, or
(c) transplanting the tumor stem cell of claim 1 or 2 into an immunocompromised mouse;
(d) administering a test compound to the immunocompromised mouse; and
(e) determining the response of the transplanted tumor stem cell to the test compound; or
(f) contacting the tumor stem cell of claim 1 or 2 with a test compound under conditions suitable to allow complex formation; and
(g) detecting complex formation between the test compound and a tumor stem cell, wherein the presence of the complex identifies the test compound as specifically binding the tumor stem cell.

6. A method for the proliferation of a tumor stem cell, comprising:
(a) proliferating the tumor stem cell of claim 1 or 2 in a culture medium, or
(b) transplanting the tumor stem cell of claim 1 or 2 into a host mammal under conditions that allow the proliferation of tumor stem cells in the host mammal, and further comprising the step of isolating the proliferated tumor stem cells from the host mammal.

7. A chimeric non-human mammal, comprising:
(a) the mammal, preferably an immunocompromised mouse; and
(b) the tumor stem cell of claim 1 or 2, wherein the cell has been injected into the mammal.

8. A method of manufacturing an anti-tumor agent comprising the steps of the method of claim 5 and synthesizing the compound tested positively for having a tumor suppressing effect in a therapeutically effective amount, optionally further comprising mixing the compound identified or isolated with a pharmaceutically acceptable carrier.

9. An anti-tumor agent identified or isolated according to the method of claim 5, which agent hitherto has not been known or used as an anti-tumor agent.

10. A solid support comprising affixed thereto nucleic acids obtained from a tumor stem cell of claim 1 or 2, preferably wherein said solid support is a microarray.

11. Kit for use in a method of any one of claims 3 to 6 or 8 comprising culture media components, growth factors, selectable markers, reference samples, microarrays, vectors, a solid support, and/or nucleic acid probes.

12. Use of a tumor promoting agent or a nucleic acid molecule encoding such tumor promoting agent for inducing a tumor in a non-human animal as a source for tumor stem cells.

13. Use of a non-human animal comprising or determined to develop a tumor due to the presence of a foreign nucleic acid molecule encoding a tumor promoting agent as a source for the isolation of tumor stem cells from said tumor.

14. The tumor stem cell of claim 1 or 2, the method of any one of claims 3 to 6, the chimeric non-human mammal of claim 7, the method of claim 8, the anti-tumor agent of claim 9, the solid support of claim 10, the kit of claim 11, or the use of claim 12 or 13, wherein the tumor is induced by the presence of a foreign nucleic acid molecule which encodes Y-box binding protein 1 (YB-1) or a tumor promoting fragment thereof.
